(19) Europäisches Patentamt
European Patent Office
Office européen des brevets

(11) **EP 4 262 555 B1**

(12) **EUROPEAN PATENT SPECIFICATION**

(45) Date of publication and mention
of the grant of the patent:
**21.01.2026 Bulletin 2026/04**

(21) Application number: **21844146.7**

(22) Date of filing: **17.12.2021**

(51) International Patent Classification (IPC):
***A61B 5/145*** (2006.01)  ***A61B 5/1486*** (2006.01)
***A61B 5/00*** (2006.01)

(52) Cooperative Patent Classification (CPC):
**A61B 5/14503; A61B 5/14532; A61B 5/14546;
A61B 5/14865; A61B 5/6833; A61B 5/685;**
A61B 2560/0223

(86) International application number:
**PCT/US2021/064208**

(87) International publication number:
**WO 2022/133313 (23.06.2022 Gazette 2022/25)**

(54) **SYSTEMS AND METHODS FOR ANALYTE DETECTION**

SYSTEME UND VERFAHREN ZUM ANALYTNACHWEIS

SYSTÈMES ET PROCÉDÉS POUR LA DÉTECTION D'ANALYTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2020 US 202063127848 P**

(43) Date of publication of application:
**25.10.2023 Bulletin 2023/43**

(73) Proprietor: **Abbott Diabetes Care, Inc.
Alameda, CA 94502 (US)**

(72) Inventor: **FELDMAN, Benjamin, J.
Berkeley, CA 94704 (US)**

(74) Representative: **Mathys & Squire
The Shard
32 London Bridge Street
London SE1 9SG (GB)**

(56) References cited:
**US-A1- 2013 144 137    US-A1- 2017 303 819
US-A1- 2020 046 269    US-A1- 2020 237 276**

**Description**

<u>PRIORITY</u>

This application claims priority to and benefit of U.S. Provisional Patent Application No. 63/127,848, filed December 18, 2020.

<u>FIELD</u>

The subject matter described herein relates to analyte sensors and methods of using the same.

<u>BACKGROUND</u>

The detection of various analytes can be used to help monitor health conditions. Detection of analytes can be used to determine, among other things, changes in analyte levels, which can be indicative of a physiological condition. For example, monitored glucose levels can be used by diabetes patients to manage their glucose levels by taking appropriate action, such as administering insulin or consuming particular foods or beverages at appropriate times based on analyte levels or trends. Other analytes can be desirable to monitor other physiological conditions, or in some instance multiple analytes can be simultaneously used to monitor multiple physiological conditions.

Monitoring analytes can occur periodically or continuously over a given period of time. For continuous monitoring, one or more sensors that remain at least partially implanted within a tissue of an individual, such as dermally, subcutaneously or intravenously, so that analyses can be conducted in vivo. Implanted sensors can collect analyte data on-demand, at a set schedule, or continuously, depending on an individual's particular health needs and/or previously measured analyte levels. For example, in vivo enzyme-based amperometric sensors can be configured to assay one or more analytes and to monitor the health of an individual. Analyte sensors can employ an enzyme having specificity or sensitivity for a particular substrate. Monitored analytes can include, for example and without limitation, glucose, lactate, oxygen, and ketones.

Periodic analyte monitoring, by comparison and without limitation, can occur by withdrawing a sample of bodily fluid, such as blood or urine, and analyzing the sample ex vivo. While ex vivo analyte monitoring can be sufficient, there are some challenges associated with ex vivo analyte monitoring. For example, withdrawing a sample can be inconvenient or painful, and the risk of lost data can be increased. Continuous analyte monitoring, including such monitoring using an *in vivo* implanted sensor, can overcome such challenges.

Another example of an analyte that can be monitored is alcohol. Information pertaining to the *in vivo* alcohol levels of an individual can be used, for example, to predict or monitor the level of another analyte of interest. For example, alcohol can alter the glycemic control of an individual, whose glucose levels are naturally dysregulated or otherwise lack homeostasis without intervention. Other analytes that can be dysregulated by alcohol can include triglycerides *(e.g.,* related to heart disease, stroke, blood pressure, obesity), gamma-glutamyl transferase **(GGT)** *(e.g.,* related to cancer, hepatitis, bone disease), and cortisol *(e.g.,* related to stress, inflammation). Alcohol monitoring can also be used, for example, to deter alcohol consumption. Accordingly, it can be helpful to continuously or periodically monitoring the alcohol level of an individual.

<u>SUMMARY</u>

US2020237276 discloses multiple enzymes present in the active area(s) of an electrochemical sensor to facilitate analyses of one or more analytes. The multiple enzymes may function independently to detect several analytes or in concert to detect a single analyte.

The purpose and advantages of the disclosed subject matter will be set forth in and are apparent from the description that follows, as well as will be learned by practice of the disclosed subject matter.

Additional advantages of the disclosed subject matter will be realized and attained by the devices particularly pointed out in the written description and claims hereof, as well as from the appended drawings. The invention is defined by an apparatus according to claim 1 and a method according to claim **8.** Further embodiments are defined by dependent claims **2-7** and **9-13.**

The time period over which the peak signal width is determined is an initial wear period of the analyte sensor. The initial wear period is, for example, 1, 2, 3, 4, or 5 days after insertion of the analyte sensor.

The one of more processors is configured to determine the blood alcohol concentration based on a ratio of a peak signal amplitude to the peak signal width.

The one or more processors is configured to determine the blood alcohol concentration based on a sensitivity of the alcohol sensor. The sensitivity of the alcohol sensor is factory set based on a calibration.

As embodied herein, the analyte sensor include one or more active areas. The active areas can include one or more enzymes, wherein the enzymes degrades over a life of the alcohol sensor. In some examples, the one or more active areas of the analyte sensor detect two or more analytes. The two or more analytes can include ethanol, glucose, or lactate.

As embodied herein, the one or more processors can display the blood alcohol concentration on the apparatus, wherein the apparatus is a reader device. In some examples, the one or more processors can include output an alert based on the blood alcohol concentration, wherein the alert is visual, auditory, or vibratory.

## BRIEF DESCRIPTION OF THE DRAWINGS

FIG. 1A is a system overview of a sensor applicator, reader device, monitoring system, network, and remote system.

FIG. 1B is a diagram illustrating an operating environment of an example analyte monitoring system for use with the techniques described herein.

FIG. 2A is a block diagram depicting an example embodiment of a reader device.

FIG. 2B is a block diagram illustrating an example data receiving device for communicating with the sensor according to exemplary embodiments of the disclosed subject matter.

FIGS. 2C and 2D are block diagrams depicting example embodiments of sensor control devices.

FIG. 2E is a block diagram illustrating an example analyte sensor according to exemplary embodiments of the disclosed subject matter.

FIG. 3A is a proximal perspective view depicting an example embodiment of a user preparing a tray for an assembly.

FIG. 3B is a side view depicting an example embodiment of a user preparing an applicator device for an assembly.

FIG. 3C is a proximal perspective view depicting an example embodiment of a user inserting an applicator device into a tray during an assembly.

FIG. 3D is a proximal perspective view depicting an example embodiment of a user removing an applicator device from a tray during an assembly.

FIG. 3E is a proximal perspective view depicting an example embodiment of a patient applying a sensor using an applicator device.

FIG. 3F is a proximal perspective view depicting an example embodiment of a patient with an applied sensor and a used applicator device.

FIG. 4A is a side view depicting an example embodiment of an applicator device coupled with a cap.

FIG. 4B is a side perspective view depicting an example embodiment of an applicator device and cap decoupled.

FIG. 4C is a perspective view depicting an example embodiment of a distal end of an applicator device and electronics housing.

FIG. 4D is a top perspective view of an exemplary applicator device in accordance with the disclosed subject matter.

FIG. 4E is a bottom perspective view of the applicator device of FIG. 4D.

FIG. 4F is an exploded view of the applicator device of FIG. 4D.

FIG. 4G is a side cutaway view of the applicator device of FIG. 4D.

FIG. 5 is a proximal perspective view depicting an example embodiment of a tray with sterilization lid coupled.

FIG. 6A is a proximal perspective cutaway view depicting an example embodiment of a tray with sensor delivery components.

FIG. 6B is a proximal perspective view depicting sensor delivery components.

FIGS. 7A and 7B are isometric exploded top and bottom views, respectively, of an exemplary sensor control device.

FIG. 8A-8C are assembly and cross-sectional views of an on-body device including an integrated connector for the sensor assembly.

FIGS. 9A and 9B are side and cross-sectional side views, respectively, of an example embodiment of the sensor applicator of FIG. 1A with the cap of FIG. 2C coupled thereto.

FIGS. 10A and 10B are isometric and side views, respectively, of another example sensor control device.

FIGS. 11A-11C are progressive cross-sectional side views showing assembly of the sensor applicator with the sensor control device of FIGS. 10A-10B.

FIGS. 12A-12C are progressive cross-sectional side views showing assembly and disassembly of an example embodiment of the sensor applicator with the sensor control device of FIGS. 10A-10B.

FIGS. 13A-13F illustrate cross-sectional views depicting an example embodiment of an applicator during a stage of deployment.

FIG. 14 is a graph depicting an example of an in vitro sensitivity of an analyte sensor.

FIG. 15 is a diagram illustrating example operational states of the sensor according to exemplary embodiments of the disclosed subject matter.

FIG. 16 is a diagram illustrating an example operational and data flow for over-the-air programming of a sensor according to the disclosed subject matter.

FIG. 17 is a diagram illustrating an example data flow for secure exchange of data between two devices according to the disclosed subject matter.

FIGS. 18A-18C are cross-sectional diagrams illustrating exemplary analyte sensors including a single active area as embodied herein.

FIG. 19 is a cross-sectional diagram illustrating an exemplary analyte sensor including two active areas as embodied herein.

FIG. 20 is a cross-sectional diagram illustrating an exemplary analyte sensor including two active areas as embodied herein.

FIGS. 21A and 21B are graphs illustrating a current output of an exemplary analyte sensor as embodied herein.

FIG. 22 is a graph illustrating a current output of an exemplary analyte sensor as embodied herein.

FIG. 23 is a graph illustrating a current output of an exemplary analyte sensor as embodied herein.

FIG. 24 is a graph illustrating a current output of an exemplary analyte sensor as embodied herein.

FIG. 25 is a diagram illustrating a correlation between the blood alcohol concentrations (BACs) measured at signal peaks and corresponding current peak widths using the exemplary analyte sensor of FIG. 24.

FIG. 26 is a diagram illustrating a correlation between the total alcohol consumptions (TACs) measured at signal peaks and corresponding current peak widths using the exemplary analyte sensor of FIG. 24.

## DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for the use of analyte sensor insertion applicators for use with in vivo analyte monitoring systems. An applicator can be provided to the user in a sterile package with an electronics housing of the sensor control device contained therein. According to some embodiments, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other embodiments, the applicator, sensor control device, sensor module, and sharp module can be provided in a single package. The applicator can be used to position the sensor control device on a human body with a sensor in contact with the wearer's bodily fluid. The embodiments provided herein are improvements to reduce the likelihood that a sensor is improperly inserted or damaged, or elicits an adverse physiological response. Other improvements and advantages are provided as well. The various configurations of these devices are described in detail by way of the embodiments which are only examples.

Furthermore, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps. These sensor control device embodiments can be used and can be capable of use to implement those steps performed by a sensor control device from any and all of the methods described herein.

Furthermore, the systems and methods presented herein can be used for operations of a sensor used in an analyte monitoring system, such as but not limited to wellness, fitness, dietary, research, information or any purposes involving analyte sensing over time. As used herein, "analyte sensor" or "sensor" can refer to any device capable of receiving sensor information from a user, including for purpose of illustration but not limited to, body temperature sensors, blood pressure sensors, pulse or heart-rate sensors, glucose level sensors, analyte sensors, physical activity sensors, body movement sensors, or any other sensors for collecting physical or biological information. Analytes measured by the analyte sensors can include, by way of example and not limitation, glucose, ketones, lactate, oxygen, hemoglobin A1C, albumin, alcohol, alkaline phosphatase, alanine transaminase, aspartate aminotransferase, bilirubin, blood urea nitrogen, calcium, carbon dioxide, chloride, creatinine, hematocrit, lactate, magnesium, oxygen, pH, phosphorus, potassium, sodium, total protein, uric acid, etc.

As mentioned, a number of embodiments of systems, devices, and methods are described herein that provide for the improved assembly and use of dermal sensor insertion devices for use with in vivo analyte monitoring systems. In particular, several embodiments of the present disclosure are designed to improve the method of sensor insertion with respect to in vivo analyte monitoring systems and, in particular, to prevent the premature retraction of an insertion sharp during a sensor insertion process. Some embodiments, for example, include a dermal sensor insertion mechanism with an increased firing velocity and a delayed sharp retraction. In other embodiments, the sharp retraction mechanism can be motion-actuated such that the sharp is not retracted until the user pulls the applicator away from the skin. Consequently, these embodiments can reduce the likelihood of prematurely withdrawing an insertion sharp during a sensor insertion process; decrease the likelihood of improper sensor insertion; and decrease the likelihood of damaging a sensor during the sensor insertion process, to name a few advantages. Several embodiments of the present disclosure also provide for improved insertion sharp modules to account for the small scale of dermal sensors and the relatively shallow insertion path present in a subject's dermal layer. In addition, several embodiments of the present disclosure are designed to prevent undesirable axial and/or rotational movement of applicator components during sensor insertion. Accordingly, these embodiments can reduce the likelihood of instability of a positioned dermal sensor, irritation at the insertion site, damage to surrounding tissue, and breakage of capillary blood vessels resulting in fouling of the dermal fluid with blood, to name a few advantages. In addition, to mitigate inaccurate sensor readings which can be caused by trauma at the insertion site, several embodiments of the present disclosure can reduce the end-depth penetration of the needle relative to the sensor tip during insertion.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

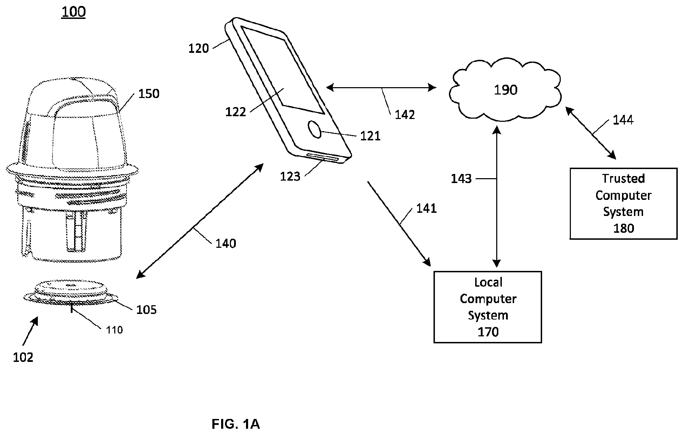

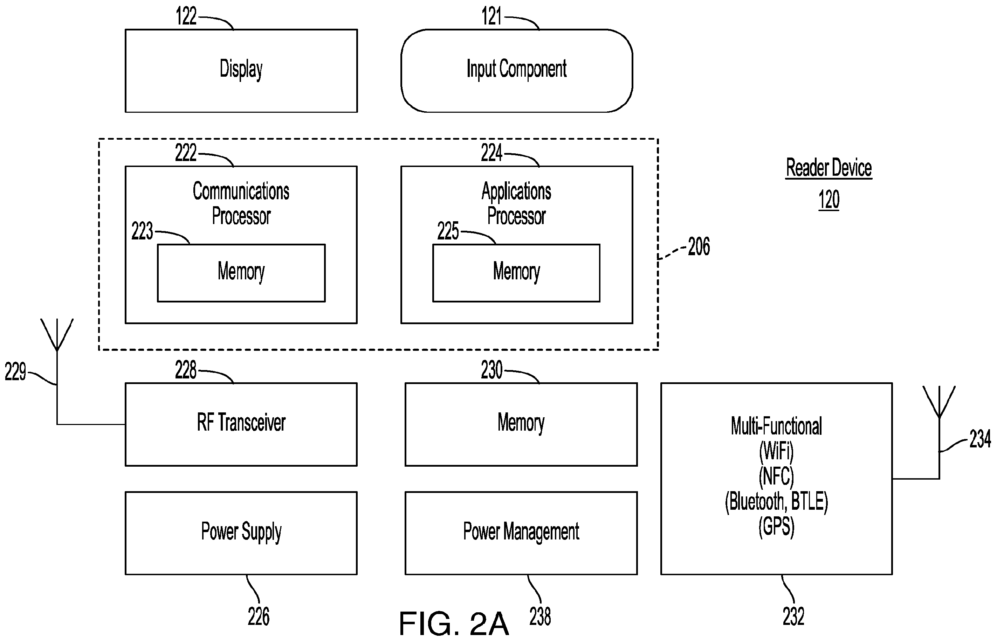

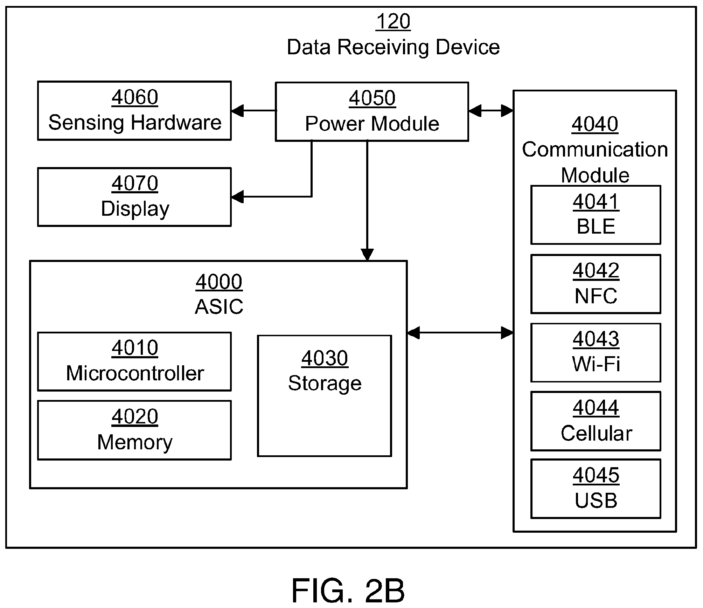

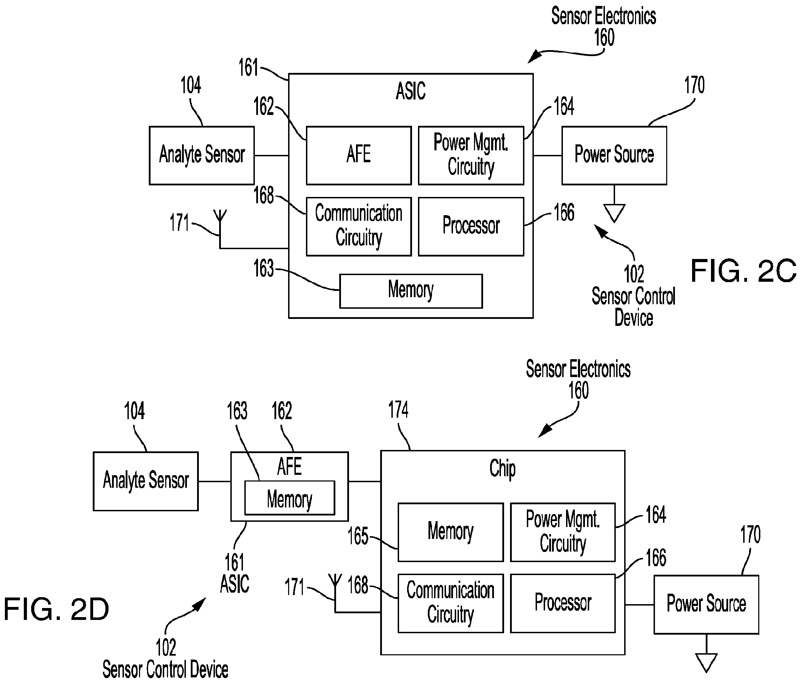

FIG. 1A is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where an analyte sensor 110 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on reader device 120 using screen 122 and input 121 and the device battery can be recharged using power port 123. More detail about reader device 120 is set forth with respect to FIG. 2A below. Reader device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

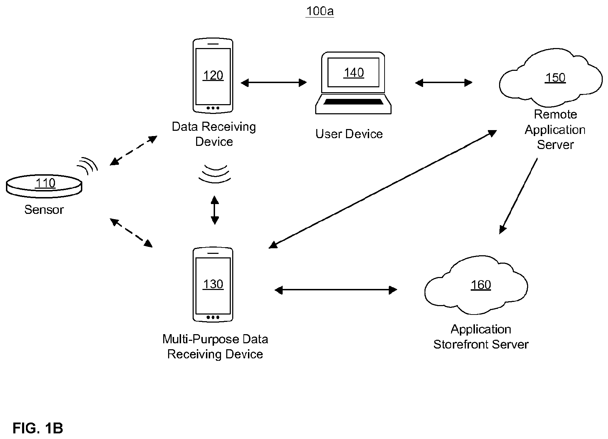

FIG. 1B illustrates an operating environment of an analyte monitoring system 100a capable of embodying the techniques described herein. The analyte monitoring system 100a can include a system of components designed to provide monitoring of parameters, such as analyte levels, of a human or animal body or can provide for other operations based on the configurations of the various components. As embodied herein, the system can include a low-power analyte sensor 110, or simply "sensor" worn by the user or attached to the body for which information is being collected. As embodied herein, the analyte sensor 110 can be a sealed, disposable device with a predetermined active use lifetime (e.g., 1 day, 14 days, 30 days, etc.). Sensors 110 can be applied to the skin of the user body and remain adhered over the duration of the sensor lifetime or can be designed to be selectively removed and remain functional when reapplied. The low-power analyte monitoring system 100a can further include a data reading device 120 or multi-purpose data receiving device 130 configured as described herein to facilitate retrieval and delivery of data, including analyte data, from the analyte sensor 110.

As embodied herein, the analyte monitoring system 100a can include a software or firmware library or application provided, for example via a remote application server 150 or application storefront server 160, to a third-party and incorporated into a multi-purpose hardware device 130 such as a mobile phone, tablet, personal computing device, or other similar computing device capable of communicating with the analyte sensor 110 over a communication link. Multi-purpose hardware can further include embedded devices, including, but not limited to insulin pumps or insulin pens, having an embedded library configured to communicate with the analyte sensor 110. Although the illustrated embodiments of the analyte monitoring system 100a include only one of each of the illustrated devices, this disclosure contemplates the analyte monitoring system 100a incorporate multiples of each components interacting throughout the system. For example and without limitation, as embodied herein, data reading device 120 and/or multi-purpose data receiving device 130 can include multiples of each. As embodied herein, multiple data receiving devices 130 can communicate directly with sensor 110 as described herein. Additionally or alternatively, a data receiving device 130 can communicate with secondary data receiving devices 130 to provide analyte data, or visualization or analysis of the data, for secondary display to the user or other authorized parties.

FIG. 2A is a block diagram depicting an example embodiment of a reader device configured as a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multifunctional transceiver 232 which can communicate over Wi-Fi, NFC, Bluetooth, BTLE, and GPS with an antenna 234. As understood by one of skill in the art, these components are electrically and communicatively coupled in a manner to make a functional device.

For purpose of illustration and not limitation, reference is made to the exemplary embodiment of a data receiving device 120 for use with the disclosed subject matter as shown in FIG. 2B. The data receiving device 120, and the related multi-purpose data receiving device 130, includes components germane to the discussion of the analyte sensor 110 and its operations and additional components can be included. In particular embodiments, the data receiving device 120 and multi-purpose data receiving device 130 can be or include components provided by a third party and are not necessarily restricted to include devices made by the same manufacturer as the sensor 110.

As illustrated in FIG. 2B, the data receiving device 120 includes an ASIC 4000 including a microcontroller 4010, memory 4020, and storage 4030 and communicatively coupled with a communication module 4040. Power for the components of the data receiving device 120 can be delivered by a power module 4050, which as embodied herein can include a rechargeable battery. The data receiving device 120 can further include a display 4070 for facilitating review of analyte data received from an analyte sensor 110 or other device (e.g., user device 140 or remote application server 150). The data receiving device 120 can include separate user interface components (e.g., physical keys, light sensors, microphones, etc.).

The communication module 4040 can include a BLE module 4041 and an NFC module 4042. The data receiving device 120 can be configured to wirelessly couple with the analyte sensor 110 and transmit commands to and receive data from the analyte sensor 110. As embodied herein, the data receiving device 120 can be configured to operate, with respect to the analyte sensor 110 as described herein, as an NFC scanner and a BLE end point via specific modules (e.g., BLE module 4042 or NFC module 4043) of the communication module 4040. For example, the data receiving device 120 can issue commands (e.g., activation commands for a data broadcast mode of the sensor; pairing commands to identify the data receiving device 120) to the analyte sensor 110 using a first module of the communication module 4040 and receive data from and transmit data to the analyte sensor 110 using a second module of the communication module 4040. The data receiving device 120 can be configured for communication with a user device 140 via a Universal Serial Bus (USB) module 4045 of the communication module 4040.

As another example, the communication module 4040 can include, for example, a cellular radio module 4044. The cellular radio module 4044 can include one or more radio transceivers for communicating using broadband cellular networks, including, but not limited to third generation (3G), fourth generation (4G), and fifth generation (5G) networks. Additionally, the communication module 4040 of the data receiving device 120 can include a Wi-Fi radio module 4043 for communication using a wireless local area network according to one or more of the IEEE 802.11 standards (e.g., 802.11a, 802.11b, 802.11g, 802.11n (aka Wi-Fi 4), 802.11ac (aka Wi-Fi 5), 802.11ax (aka Wi-Fi 6)). Using the cellular radio module 4044 or Wi-Fi radio module 4043, the data receiving device 120 can communicate with the remote application server 150 to receive analyte data or provide updates or input received from a user (e.g., through one or more user interfaces). Although not illustrated, the communication module 5040 of the analyte sensor 120 can similarly include a cellular radio module or Wi-Fi radio module.

As embodied herein, the on-board storage 4030 of the data receiving device 120 can store analyte data received from the analyte sensor 110. Further, the data receiving device 120, multi-purpose data receiving device 130, or a user device 140 can be configured to communicate with a remote application server 150 via a wide area network. As embodied herein, the analyte sensor 110 can provide data to the data receiving device 120 or multi-purpose data receiving device 130. The data receiving device 120 can transmit the data to the user computing device 140. The user computing device 140 (or the multi-purpose data receiving device 130) can in turn transmit that data to a remote application server 150 for processing and analysis.

As embodied herein, the data receiving device 120 can further include sensing hardware 4060 similar to, or expanded from, the sensing hardware 5060 of the analyte sensor 110. In particular embodiments, the data receiving device 120 can be configured to operate in coordination with the analyte sensor 110 and based on analyte data received from the analyte sensor 110. As an example, where the analyte sensor 110 glucose sensor, the data receiving device 120 can be or include an insulin pump or insulin injection pen. In coordination, the compatible device 130 can adjust an insulin dosage for a user based on glucose values received from the analyte sensor.

FIGS. 2C and 2D are block diagrams depicting example embodiments of sensor control device 102 having analyte sensor 110 and sensor electronics 160 (including analyte monitoring circuitry) that can have the majority of the processing capability for rendering end-result data suitable for display to the user. In FIG. 2C, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or nonvolatile memory. In this embodiment, ASIC 161 is coupled with power source 170, which can be a coin cell battery, or the like. AFE 162 interfaces with in vivo analyte sensor 110 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 2D is similar to FIG. 2C but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

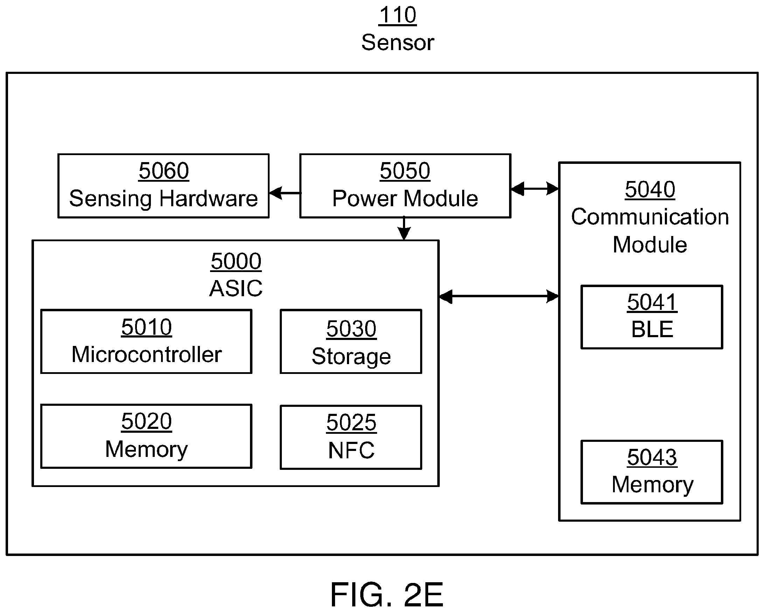

For purpose of illustration and not limitation, reference is made to the exemplary embodiment of an analyte sensor 110 for use with the disclosed subject matter as shown in FIG. 2E. FIG. 2E illustrates a block diagram of an example analyte sensor 110 according to exemplary embodiments compatible with the security architecture and communication schemes described herein.

As embodied herein, the analyte sensor 110 can include an Application-Specific Integrated Circuit ("ASIC") 5000 communicatively coupled with a communication module 5040. The ASIC 5000 can include a microcontroller core 5010, on-board memory 5020, and storage memory 5030. The storage memory 5030 can store data used in an authentication and encryption security architecture. The storage memory 5030 can store programming instructions for the sensor 110. As embodied herein, certain communication chipsets can be embedded in the ASIC 5000 (e.g., an NFC transceiver 5025). The ASIC 5000 can receive power from a power module 5050, such as an on-board battery or from an NFC pulse. The storage memory 5030 of the ASIC 5000 can be programmed to include information such as an identifier for the sensor 110 for identification and tracking purposes. The storage memory 5030 can also be programmed with configuration or calibration parameters for use by the sensor 110 and its various components. The storage memory 5030 can include rewritable or one-time programming (OTP) memory. The storage memory 5030 can be updated using techniques described herein to extend the usefulness of the sensor 110.

As embodied herein, the communication module 5040 of the sensor 100 can be or include one or more modules to support the analyte sensor 110 communicating with other devices of the analyte monitoring system 100. As an example only and not by way of limitation, example communication modules 5040 can include a Bluetooth Low-Energy ("BLE") module 5041 As used throughout this disclosure, Bluetooth Low Energy ("BLE") refers to a short-range communication protocol optimized to make pairing of Bluetooth devices simple for end users. The communication module 5040 can transmit and receive data and commands via interaction with similarly-capable communication modules of a data receiving device 120 or user device 140. The communication module 5040 can include additional or alternative chipsets for use with similar short-range communication schemes, such as a personal area network according to IEEE 802.15 protocols, IEEE 802.11 protocols, infrared communications according to the Infrared Data Association standards (IrDA), etc.

To perform its functionalities, the sensor 100 can further include suitable sensing hardware 5060 appropriate to its function. As embodied herein, the sensing hardware 5060 can include an analyte sensor transcutaneously or subcutaneously positioned in contact with a bodily fluid of a subject. The analyte sensor can generate sensor data containing values corresponding to levels of one or more analytes within the bodily fluid.

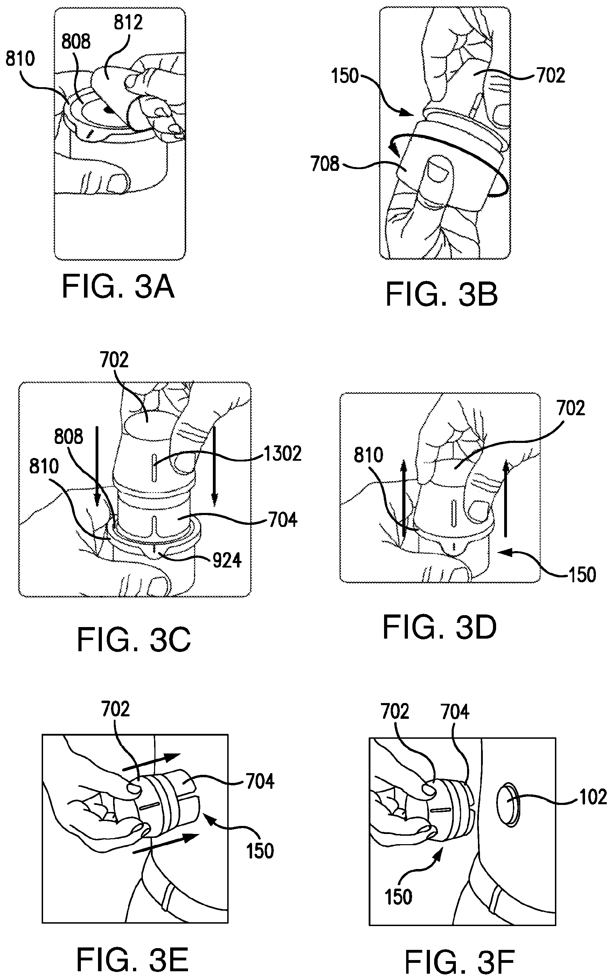

The components of sensor control device 102 can be acquired by a user in multiple packages requiring final assembly by the user before delivery to an appropriate user location. FIGS. 3A-3D depict an example embodiment of an assembly process for sensor control device 102 by a user, including preparation of separate components before coupling the components in order to ready the sensor for delivery. FIGS. 3E-3F depict an example embodiment of delivery of sensor control device 102 to an appropriate user location by selecting the appropriate delivery location and applying device 102 to the location.

FIG. 3A is a proximal perspective view depicting an example embodiment of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various embodiments so long as platform 808 is adequately exposed within tray 810. Lid 812 can then be placed aside.

FIG. 3B is a side view depicting an example embodiment of a user preparing an applicator device 150 for assembly. Applicator device 150 can be provided in a sterile package sealed by a cap 708. Preparation of applicator device 150 can include uncoupling housing 702 from cap 708 to expose sheath 704 (FIG. 3C). This can be accomplished by unscrewing (or otherwise uncoupling) cap 708 from housing 702. Cap 708 can then be placed aside.

FIG. 3C is a proximal perspective view depicting an example embodiment of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This step unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example embodiment of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example embodiment of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example embodiment of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

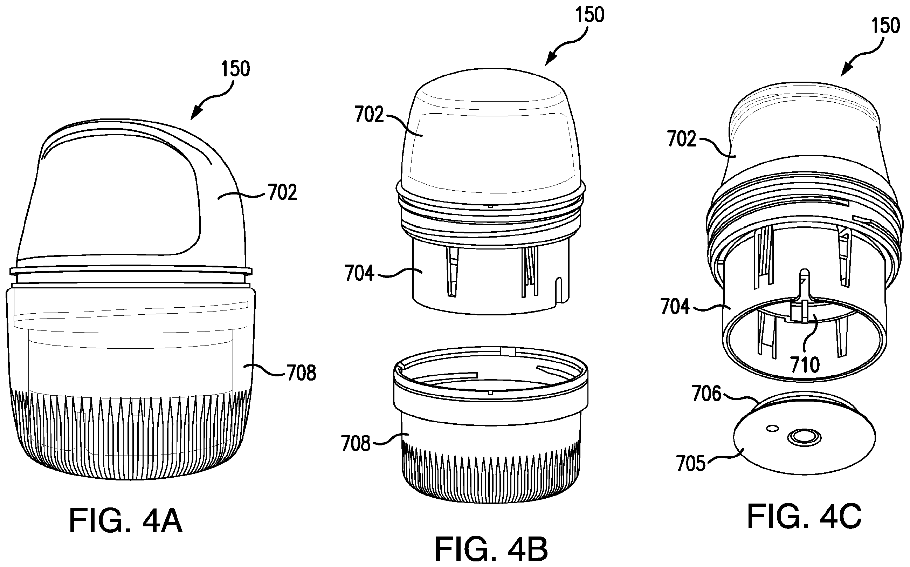

FIG. 4A is a side view depicting an example embodiment of an applicator device 150 coupled with screw cap 708. This is an example of how applicator 150 is shipped to and received by a user, prior to assembly by the user with a sensor. FIG. 4B is a side perspective view depicting applicator 150 and cap 708 after being decoupled. FIG. 4C is a perspective view depicting an example embodiment of a distal end of an applicator device 150 with electronics housing 706 and adhesive patch 105 removed from the position they would have retained within sensor carrier 710 of sheath 704, when cap 708 is in place.

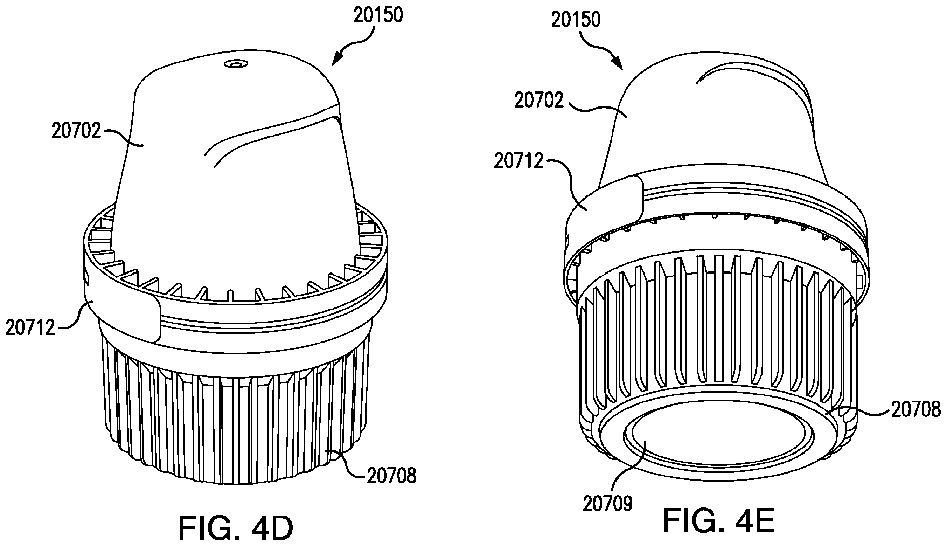

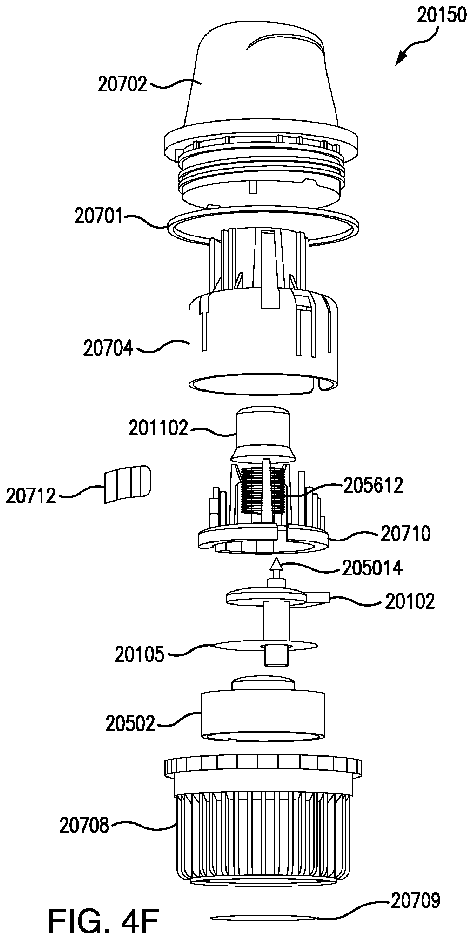

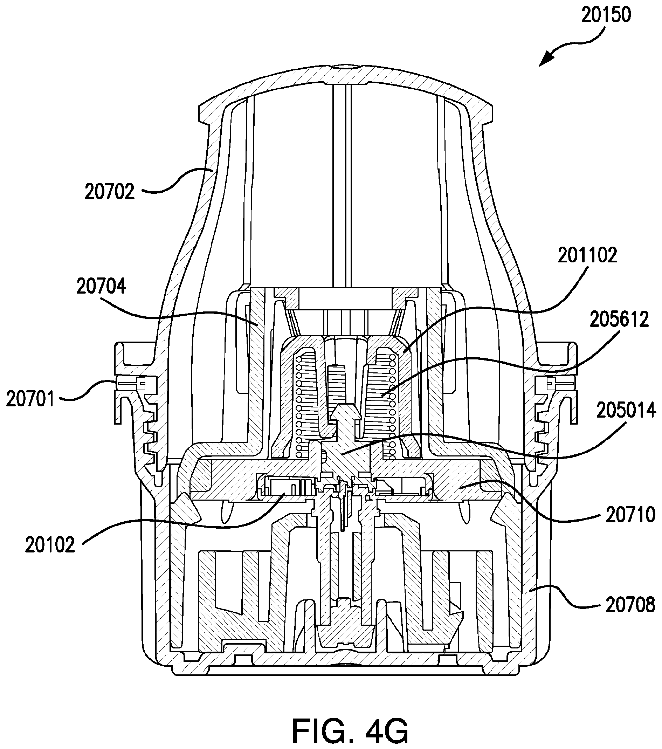

Referring to FIG. 4D-G for purpose of illustration and not limitation, the applicator device 20150 can be provided to a user as a single integrated assembly. FIGs. 4D and 4E provide perspective top and bottom views, respectively, of the applicator device 20150, FIG. 4F provides an exploded view of the applicator device 20150 and FIG. 4G provides a side cut-away view. The perspective views illustrate how applicator 20150 is shipped to and received by a user. The exploded and cut-away views illustrate the components of the applicator device 20150. The applicator device 20150 can include a housing 20702, gasket 20701, sheath 20704, sharp carrier 201102, spring 205612, sensor carrier 20710 (also referred to as a "puck carrier"), sharp hub 205014, sensor control device (also referred to as a "puck") 20102, adhesive patch 20105, desiccant 20502, cap 20708, serial label 20709, and tamper evidence feature 20712. As received by a user, only the housing 20702, cap 20708, tamper evidence feature 20712, and label 20709 are visible. The tamper evidence feature 20712 can be, for example, a sticker coupled to each of the housing 20702 and the cap 20708, and tamper evidence feature 20712 can be damaged, for example, irreparably, by uncoupling housing 20702 and cap 20708, thereby indicating to a user that the housing 20702 and cap 20708 have been previously uncoupled. These features are described in greater detail below.

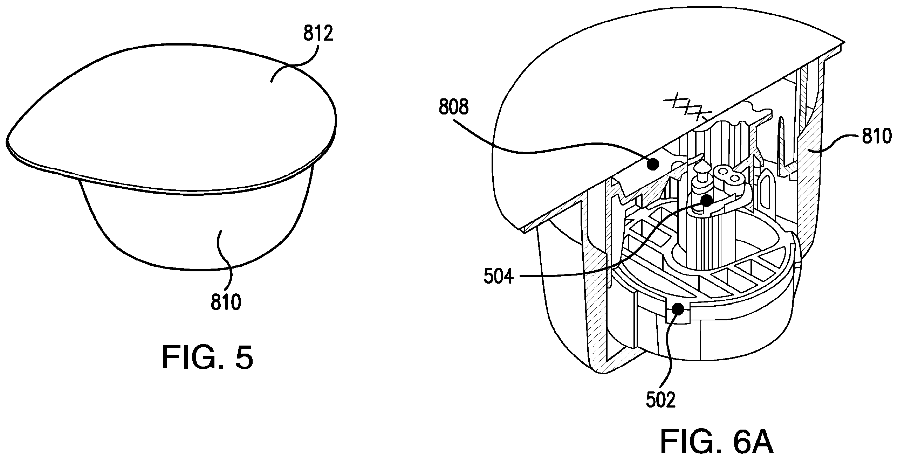

FIG. 5 is a proximal perspective view depicting an example embodiment of a tray 810 with sterilization lid 812 removably coupled thereto, which may be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

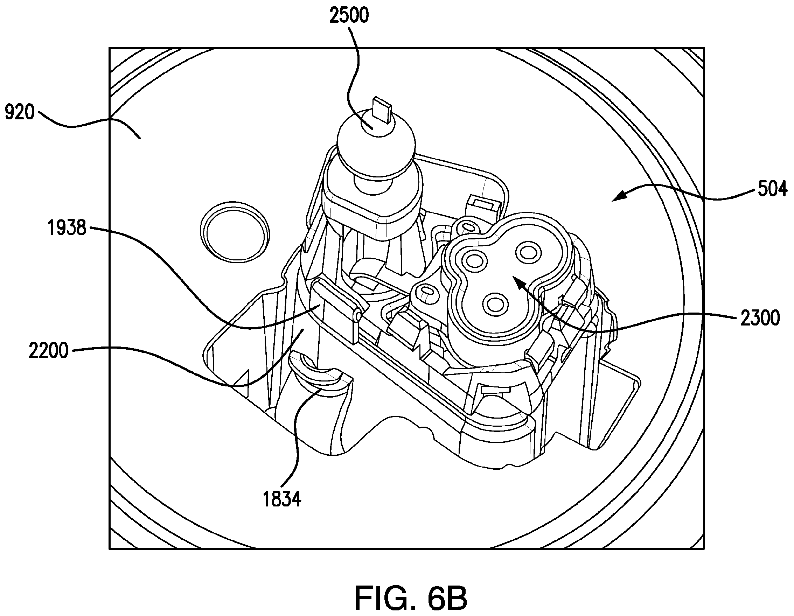

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

Referring briefly again to FIGS. 1 and 3A-3G, for the two-piece architecture system, the sensor tray 202 and the sensor applicator 102 are provided to the user as separate packages, thus requiring the user to open each package and finally assemble the system. In some applications, the discrete, sealed packages allow the sensor tray 202 and the sensor applicator 102 to be sterilized in separate sterilization processes unique to the contents of each package and otherwise incompatible with the contents of the other. More specifically, the sensor tray 202, which includes the plug assembly 207, including the sensor 110 and the sharp 220, may be sterilized using radiation sterilization, such as electron beam (or "e-beam") irradiation. Suitable radiation sterilization processes include, but are not limited to, electron beam (e-beam) irradiation, gamma ray irradiation, X-ray irradiation, or any combination thereof. Radiation sterilization, however, can damage the electrical components arranged within the electronics housing of the sensor control device 102. Consequently, if the sensor applicator 102, which contains the electronics housing of the sensor control device 102, needs to be sterilized, it may be sterilized via another method, such as gaseous chemical sterilization using, for example, ethylene oxide. Gaseous chemical sterilization, however, can damage the enzymes or other chemistry and biologies included on the sensor 110. Because of this sterilization incompatibility, the sensor tray 202 and the sensor applicator 102 are commonly sterilized in separate sterilization processes and subsequently packaged separately, which requires the user to finally assemble the components for use.

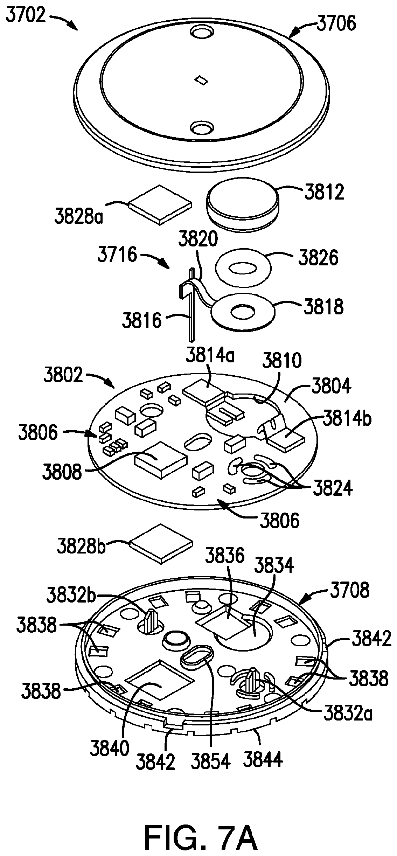

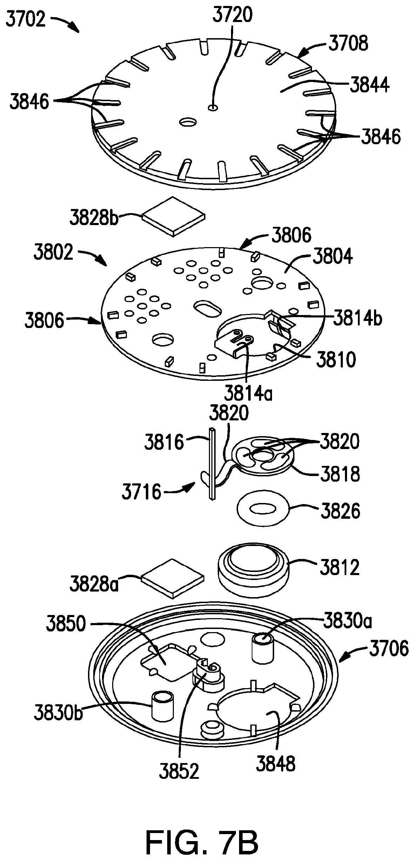

FIGS. 7A and 7B are exploded top and bottom views, respectively, of the sensor control device 3702, according to one or more embodiments. The shell 3706 and the mount 3708 operate as opposing clamshell halves that enclose or otherwise substantially encapsulate the various electronic components of the sensor control device 3702. As illustrated, the sensor control device 3702 may include a printed circuit board assembly (PCBA) 3802 that includes a printed circuit board (PCB) 3804 having a plurality of electronic modules 3806 coupled thereto. Example electronic modules 3806 include, but are not limited to, resistors, transistors, capacitors, inductors, diodes, and switches. Prior sensor control devices commonly stack PCB components on only one side of the PCB. In contrast, the PCB components 3806 in the sensor control device 3702 can be dispersed about the surface area of both sides (i.e., top and bottom surfaces) of the PCB 3804.

Besides the electronic modules 3806, the PCBA 3802 may also include a data processing unit 3808 mounted to the PCB 3804. The data processing unit 3808 may comprise, for example, an application specific integrated circuit (ASIC) configured to implement one or more functions or routines associated with operation of the sensor control device 3702. More specifically, the data processing unit 3808 may be configured to perform data processing functions, where such functions may include but are not limited to, filtering and encoding of data signals, each of which corresponds to a sampled analyte level of the user. The data processing unit 3808 may also include or otherwise communicate with an antenna for communicating with the reader device 106 (FIG. 1).

A battery aperture 3810 may be defined in the PCB 3804 and sized to receive and seat a battery 3812 configured to power the sensor control device 3702. An axial battery contact 3814a and a radial battery contact 3814b may be coupled to the PCB 3804 and extend into the battery aperture 3810 to facilitate transmission of electrical power from the battery 3812 to the PCB 3804. As their names suggest, the axial battery contact 3814a may be configured to provide an axial contact for the battery 3812, while the radial battery contact 3814b may provide a radial contact for the battery 3812. Locating the battery 3812 within the battery aperture 3810 with the battery contacts 3814a,b helps reduce the height H of the sensor control device 3702, which allows the PCB 3804 to be located centrally and its components to be dispersed on both sides (i.e., top and bottom surfaces). This also helps facilitate the chamfer 3718 provided on the electronics housing 3704.

The sensor 3716 may be centrally located relative to the PCB 3804 and include a tail 3816, a flag 3818, and a neck 3820 that interconnects the tail 3816 and the flag 3818. The tail 3816 may be configured to extend through the central aperture 3720 of the mount 3708 to be transcutaneously received beneath a user's skin. Moreover, the tail 3816 may have an enzyme or other chemistry included thereon to help facilitate analyte monitoring.

The flag 3818 may include a generally planar surface having one or more sensor contacts 3822 (three shown in FIG. 7B) arranged thereon. The sensor contact(s) 3822 may be configured to align with and engage a corresponding one or more circuitry contacts 3824 (three shown in FIG. 7A) provided on the PCB 3804. In some embodiments, the sensor contact(s) 3822 may comprise a carbon impregnated polymer printed or otherwise digitally applied to the flag 3818. Prior sensor control devices typically include a connector made of silicone rubber that encapsulates one or more compliant carbon impregnated polymer modules that serve as electrical conductive contacts between the sensor and the PCB. In contrast, the presently disclosed sensor contacts(s) 3822 provide a direct connection between the sensor 3716 and the PCB 3804 connection, which eliminates the need for the prior art connector and advantageously reduces the height H. Moreover, eliminating the compliant carbon impregnated polymer modules eliminates a significant circuit resistance and therefor improves circuit conductivity.

The sensor control device 3702 may further include a compliant member 3826, which may be arranged to interpose the flag 3818 and the inner surface of the shell 3706. More specifically, when the shell 3706 and the mount 3708 are assembled to one another, the compliant member 3826 may be configured to provide a passive biasing load against the flag 3818 that forces the sensor contact(s) 3822 into continuous engagement with the corresponding circuitry contact(s) 3824. In the illustrated embodiment, the compliant member 3826 is an elastomeric O-ring, but could alternatively comprise any other type of biasing device or mechanism, such as a compression spring or the like, without departing from the scope of the disclosure.

The sensor control device 3702 may further include one or more electromagnetic shields, shown as a first shield 3828a and a second shield The shell 3706 may provide or otherwise define a first clocking receptacle 3830a (FIG. 7B) and a second clocking receptacle 3830b (FIG. 7B), and the mount 3708 may provide or otherwise define a first clocking post 3832a (FIG. 7A) and a second clocking post 3832b (FIG. 7A). Mating the first and second clocking receptacles 3830a,b with the first and second clocking posts 3832a,b, respectively, will properly align the shell 3706 to the mount 3708.

Referring specifically to FIG. 7A, the inner surface of the mount 3708 may provide or otherwise define a plurality of pockets or depressions configured to accommodate various component parts of the sensor control device 3702 when the shell 3706 is mated to the mount 3708. For example, the inner surface of the mount 3708 may define a battery locator 3834 configured to accommodate a portion of the battery 3812 when the sensor control device 3702 is assembled. An adjacent contact pocket 3836 may be configured to accommodate a portion of the axial contact 3814a.

Moreover, a plurality of module pockets 3838 may be defined in the inner surface of the mount 3708 to accommodate the various electronic modules 3806 arranged on the bottom of the PCB 3804. Furthermore, a shield locator 3840 may be defined in the inner surface of the mount 3708 to accommodate at least a portion of the second shield 3828b when the sensor control device 3702 is assembled. The battery locator 3834, the contact pocket 3836, the module pockets 3838, and the shield locator 3840 all extend a short distance into the inner surface of the mount 3708 and, as a result, the overall height H of the sensor control device 3702 may be reduced as compared to prior sensor control devices. The module pockets 3838 may also help minimize the diameter of the PCB 3804 by allowing PCB components to be arranged on both sides (i.e., top and bottom surfaces).

Still referring to FIG. 7A, the mount 3708 may further include a plurality of carrier grip features 3842 (two shown) defined about the outer periphery of the mount 3708. The carrier grip features 3842 are axially offset from the bottom 3844 of the mount 3708, where a transfer adhesive (not shown) may be applied during assembly. In contrast to prior sensor control devices, which commonly include conical carrier grip features that intersect with the bottom of the mount, the presently disclosed carrier grip features 3842 are offset from the plane (i.e., the bottom 3844) where the transfer adhesive is applied. This may prove advantageous in helping ensure that the delivery system does not inadvertently stick to the transfer adhesive during assembly. Moreover, the presently disclosed carrier grip features 3842 eliminate the need for a scalloped transfer adhesive, which simplifies the manufacture of the transfer adhesive and eliminates the need to accurately clock the transfer adhesive relative to the mount 3708. This also increases the bond area and, therefore, the bond strength.

Referring to FIG. 7B, the bottom 3844 of the mount 3708 may provide or otherwise define a plurality of grooves 3846, which may be defined at or near the outer periphery of the mount 3708 and equidistantly spaced from each other. A transfer adhesive (not shown) may be coupled to the bottom 3844 and the grooves 3846 may be configured to help convey (transfer) moisture away from the sensor control device 3702 and toward the periphery of the mount 3708 during use. In some embodiments, the spacing of the grooves 3846 may interpose the module pockets 3838 (FIG. 7A) defined on the opposing side (inner surface) of the mount 3708. As will be appreciated, alternating the position of the grooves 3846 and the module pockets 3838 ensures that the opposing features on either side of the mount 3708 do not extend into each other. This may help maximize usage of the material for the mount 3708 and thereby help maintain a minimal height H of the sensor control device 3702. The module pockets 3838 may also significantly reduce mold sink, and improve the flatness of the bottom 3844 that the transfer adhesive bonds to.

Still referring to FIG. 7B, the inner surface of the shell 3706 may also provide or otherwise define a plurality of pockets or depressions configured to accommodate various component parts of the sensor control device 3702 when the shell 3706 is mated to the mount 3708. For example, the inner surface of the shell 3706 may define an opposing battery locator 3848 arrangeable opposite the battery locator 3834 (FIG. 7A) of the mount 3708 and configured to accommodate a portion of the battery 3812 when the sensor control device 3702 is assembled. The opposing battery locator 3848 extends a short distance into the inner surface of the shell 3706, which helps reduce the overall height H of the sensor control device 3702.

A sharp and sensor locator 3852 may also be provided by or otherwise defined on the inner surface of the shell 3706. The sharp and sensor locator 3852 may be configured to receive both the sharp (not shown) and a portion of the sensor 3716. Moreover, the sharp and sensor locator 3852 may be configured to align and/or mate with a corresponding sharp and sensor locator 2054 (FIG. 7A) provided on the inner surface of the mount 3708.

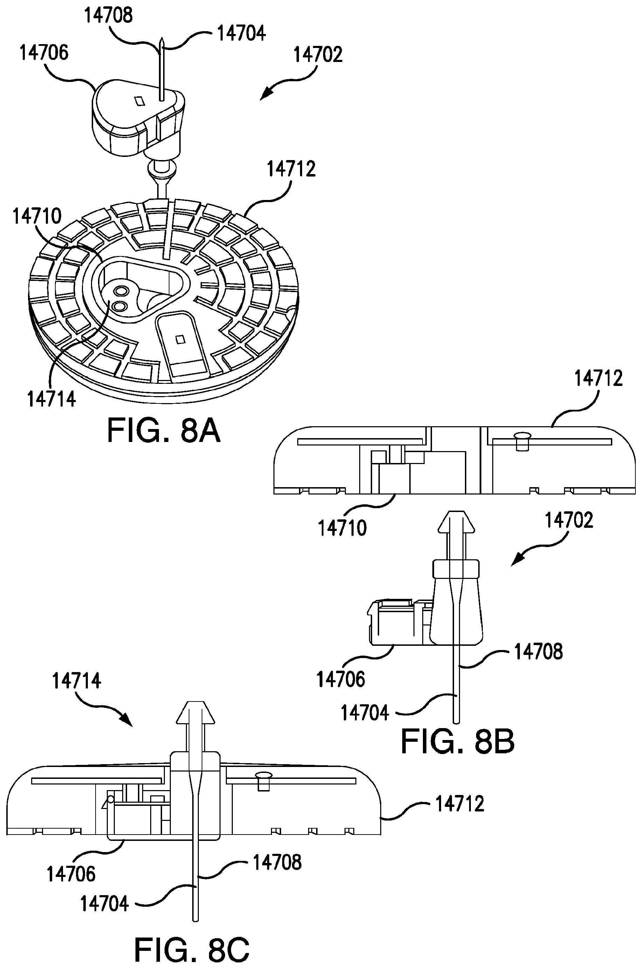

According to embodiments of the present disclosure, an alternative sensor assembly/electronics assembly connection approach is illustrated in FIGS. 8A to 8C. As shown, the sensor assembly 14702 includes sensor 14704, connector support 14706, and sharp 14708. Notably, a recess or receptacle 14710 may be defined in the bottom of the mount of the electronics assembly 14712 and provide a location where the sensor assembly 14702 may be received and coupled to the electronics assembly 14712, and thereby fully assemble the sensor control device. The profile of the sensor assembly 14702 may match or be shaped in complementary fashion to the receptacle 14710, which includes an elastomeric sealing member 14714 (including conductive material coupled to the circuit board and aligned with the electrical contacts of the sensor 14704). Thus, when the sensor assembly 14702 is snap fit or otherwise adhered to the electronics assembly 14712 by driving the sensor assembly 14702 into the integrally formed recess 14710 in the electronics assembly 14712, the on-body device 14714 depicted in FIG. 8C is formed. This embodiment provides an integrated connector for the sensor assembly 14702 within the electronics assembly 14712.

Additional information regarding sensor assemblies is provided in U.S. Publication No. 2013/0150691 and U.S. Publication No. 2021/0204841.

According to embodiments of the present disclosure, the sensor control device 102 may be modified to provide a one-piece architecture that may be subjected to sterilization techniques specifically designed for a one-piece architecture sensor control device. A one-piece architecture allows the sensor applicator 150 and the sensor control device 102 to be shipped to the user in a single, sealed package that does not require any final user assembly steps. Rather, the user need only open one package and subsequently deliver the sensor control device 102 to the target monitoring location. The one-piece system architecture described herein may prove advantageous in eliminating component parts, various fabrication process steps, and user assembly steps. As a result, packaging and waste are reduced, and the potential for user error or contamination to the system is mitigated.

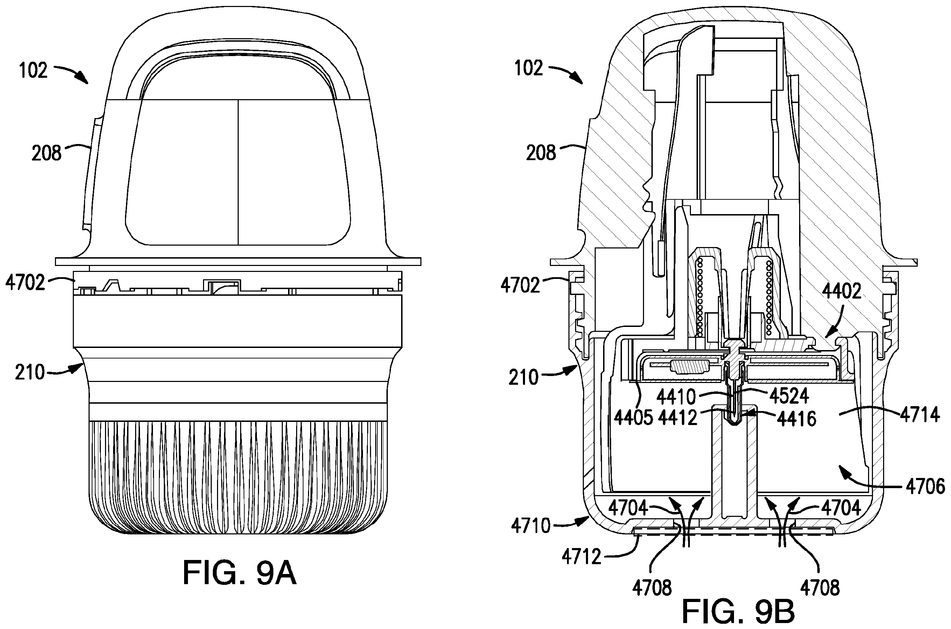

FIGS. 9A and 9B are side and cross-sectional side views, respectively, of an example embodiment of the sensor applicator 102 with the applicator cap 210 coupled thereto. More specifically, FIG. 9A depicts how the sensor applicator 102 might be shipped to and received by a user, and FIG. 9B depicts the sensor control device 4402 arranged within the sensor applicator 102. Accordingly, the fully assembled sensor control device 4402 may already be assembled and installed within the sensor applicator 102 prior to being delivered to the user, thus removing any additional assembly steps that a user would otherwise have to perform.

The fully assembled sensor control device 4402 may be loaded into the sensor applicator 102, and the applicator cap 210 may subsequently be coupled to the sensor applicator 102. In some embodiments, the applicator cap 210 may be threaded to the housing 208 and include a tamper ring 4702. Upon rotating (e.g., unscrewing) the applicator cap 210 relative to the housing 208, the tamper ring 4702 may shear and thereby free the applicator cap 210 from the sensor applicator 102.

According to the present disclosure, while loaded in the sensor applicator 102, the sensor control device 4402 may be subjected to gaseous chemical sterilization 4704 configured to sterilize the electronics housing 4404 and any other exposed portions of the sensor control device 4402. To accomplish this, a chemical may be injected into a sterilization chamber 4706 cooperatively defined by the sensor applicator 102 and the interconnected cap 210. In some applications, the chemical may be injected into the sterilization chamber 4706 via one or more vents 4708 defined in the applicator cap 210 at its proximal end 610. Example chemicals that may be used for the gaseous chemical sterilization 4704 include, but are not limited to, ethylene oxide, vaporized hydrogen peroxide, nitrogen oxide (e.g., nitrous oxide, nitrogen dioxide, etc.), and steam.

Since the distal portions of the sensor 4410 and the sharp 4412 are sealed within the sensor cap 4416, the chemicals used during the gaseous chemical sterilization process do not interact with the enzymes, chemistry, and biologics provided on the tail 4524 and other sensor components, such as membrane coatings that regulate analyte influx.

Once a desired sterility assurance level has been achieved within the sterilization chamber 4706, the gaseous solution may be removed and the sterilization chamber 4706 may be aerated. Aeration may be achieved by a series of vacuums and subsequently circulating a gas (e.g., nitrogen) or filtered air through the sterilization chamber 4706. Once the sterilization chamber 4706 is properly aerated, the vents 4708 may be occluded with a seal 4712 (shown in dashed lines).

In some embodiments, the seal 4712 may comprise two or more layers of different materials. The first layer may be made of a synthetic material (e.g., a flash-spun high-density polyethylene fiber), such as Tyvek® available from DuPont®. Tyvek® is highly durable and puncture resistant and allows the permeation of vapors. The Tyvek® layer can be applied before the gaseous chemical sterilization process, and following the gaseous chemical sterilization process, a foil or other vapor and moisture resistant material layer may be sealed (e.g., heat sealed) over the Tyvek® layer to prevent the ingress of contaminants and moisture into the sterilization chamber 4706. In other embodiments, the seal 4712 may comprise only a single protective layer applied to the applicator cap 210. In such embodiments, the single layer may be gas permeable for the sterilization process, but may also be capable of protection against moisture and other harmful elements once the sterilization process is complete.

With the seal 4712 in place, the applicator cap 210 provides a barrier against outside contamination, and thereby maintains a sterile environment for the assembled sensor control device 4402 until the user removes (unthreads) the applicator cap 210. The applicator cap 210 may also create a dust-free environment during shipping and storage that prevents the adhesive patch 4714 from becoming dirty.

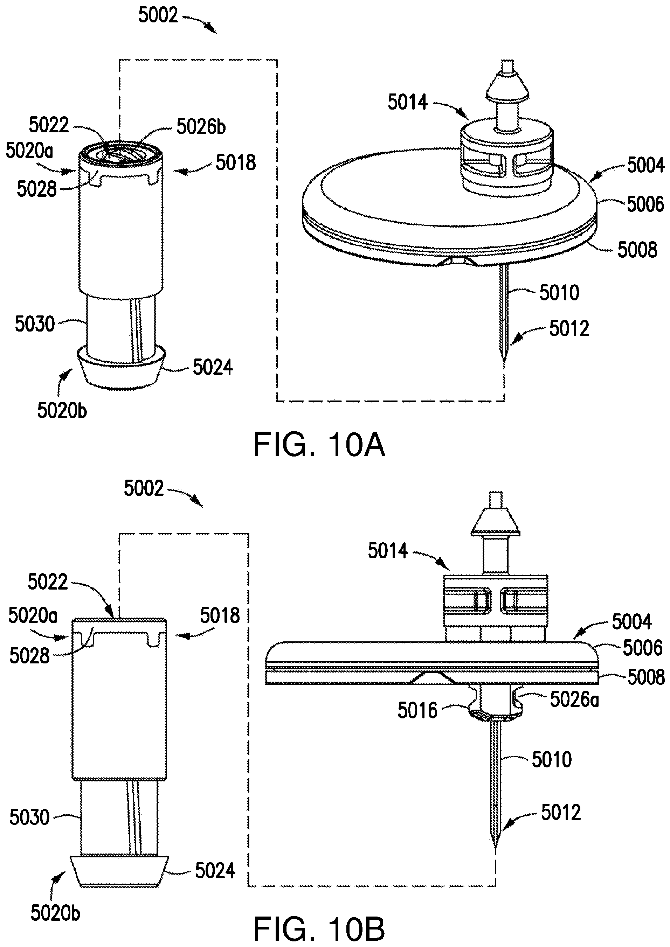

FIGS. 10A and 10B are isometric and side views, respectively, of another example sensor control device 5002, according to one or more embodiments of the present disclosure. The sensor control device 5002 may be similar in some respects to the sensor control device 102 of FIG. 1 and therefore may be best understood with reference thereto. Moreover, the sensor control device 5002 may replace the sensor control device 102 of FIG. 1 and, therefore, may be used in conjunction with the sensor applicator 102 of FIG. 1, which may deliver the sensor control device 5002 to a target monitoring location on a user's skin.

Unlike the sensor control device 102 of FIG. 1, however, the sensor control device 5002 may comprise a one-piece system architecture not requiring a user to open multiple packages and finally assemble the sensor control device 5002 prior to application. Rather, upon receipt by the user, the sensor control device 5002 may already be fully assembled and properly positioned within the sensor applicator 150 (FIG. 1). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and may have a circular cross-section. In other embodiments, however, the electronics housing 5004 may exhibit other cross-sectional shapes, such as ovoid or polygonal, without departing from the scope of the disclosure. The electronics housing 5004 may be configured to house or otherwise contain various electrical components used to operate the sensor control device 5002. In at least one embodiment, an adhesive patch (not shown) may be arranged at the bottom of the electronics housing 5004. The adhesive patch may be similar to the adhesive patch 105 of FIG. 1, and may thus help adhere the sensor control device 5002 to the user's skin for use.

As illustrated, the sensor control device 5002 includes an electronics housing 5004 that includes a shell 5006 and a mount 5008 that is matable with the shell 5006. The shell 5006 may be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 may be secured to the mount 5008 such that a sealed interface is generated therebetween.

The sensor control device 5002 may further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 may include a sharp hub 5014 configured to secure and carry the sharp 5012. As best seen in FIG. 10B, the sharp hub 5014 may include or otherwise define a mating member 5016. To couple the sharp 5012 to the sensor control device 5002, the sharp 5012 may be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 may be received within a hollow or recessed (arcuate) portion of the sharp 5012. The remaining portion of the sensor 5010 is arranged within the interior of the electronics housing 5004.

The sensor control device 5002 may further include a sensor cap 5018, shown exploded or detached from the electronics housing 5004 in FIGS. 10A-10B. The sensor cap 5016 may be removably coupled to the sensor control device 5002 (e.g., the electronics housing 5004) at or near the bottom of the mount 5008. The sensor cap 5018 may help provide a sealed barrier that surrounds and protects the exposed portions of the sensor 5010 and the sharp 5012 from gaseous chemical sterilization. As illustrated, the sensor cap 5018 may comprise a generally cylindrical body having a first end 5020a and a second end 5020b opposite the first end 5020a. The first end 5020a may be open to provide access into an inner chamber 5022 defined within the body. In contrast, the second end 5020b may be closed and may provide or otherwise define an engagement feature 5024. As described herein, the engagement feature 5024 may help mate the sensor cap 5018 to the cap (e.g., the applicator cap 708 of FIG. 3B) of a sensor applicator (e.g., the sensor applicator 150 of FIGS. 1 and 3A-3G), and may help remove the sensor cap 5018 from the sensor control device 5002 upon removing the cap from the sensor applicator.

The sensor cap 5018 may be removably coupled to the electronics housing 5004 at or near the bottom of the mount 5008. More specifically, the sensor cap 5018 may be removably coupled to the mating member 5016, which extends distally from the bottom of the mount 5008. In at least one embodiment, for example, the mating member 5016 may define a set of external threads 5026a (FIG. 10B) matable with a set of internal threads 5026b (FIG. 10A) defined by the sensor cap 5018. In some embodiments, the external and internal threads 5026a, b may comprise a flat thread design (e.g., lack of helical curvature), which may prove advantageous in molding the parts. Alternatively, the external and internal threads 5026a,b may comprise a helical threaded engagement. Accordingly, the sensor cap 5018 may be threadably coupled to the sensor control device 5002 at the mating member 5016 of the sharp hub 5014. In other embodiments, the sensor cap 5018 may be removably coupled to the mating member 5016 via other types of engagements including, but not limited to, an interference or friction fit, or a frangible member or substance that may be broken with minimal separation force (e.g., axial or rotational force).

In some embodiments, the sensor cap 5018 may comprise a monolithic (singular) structure extending between the first and second ends 5020a, b. In other embodiments, however, the sensor cap 5018 may comprise two or more component parts. In the illustrated embodiment, for example, the sensor cap 5018 may include a seal ring 5028 positioned at the first end 5020a and a desiccant cap 5030 arranged at the second end 5020b. The seal ring 5028 may be configured to help seal the inner chamber 5022, as described in more detail below. In at least one embodiment, the seal ring 5028 may comprise an elastomeric O-ring. The desiccant cap 5030 may house or comprise a desiccant to help maintain preferred humidity levels within the inner chamber 5022. The desiccant cap 5030 may also define or otherwise provide the engagement feature 5024 of the sensor cap 5018.

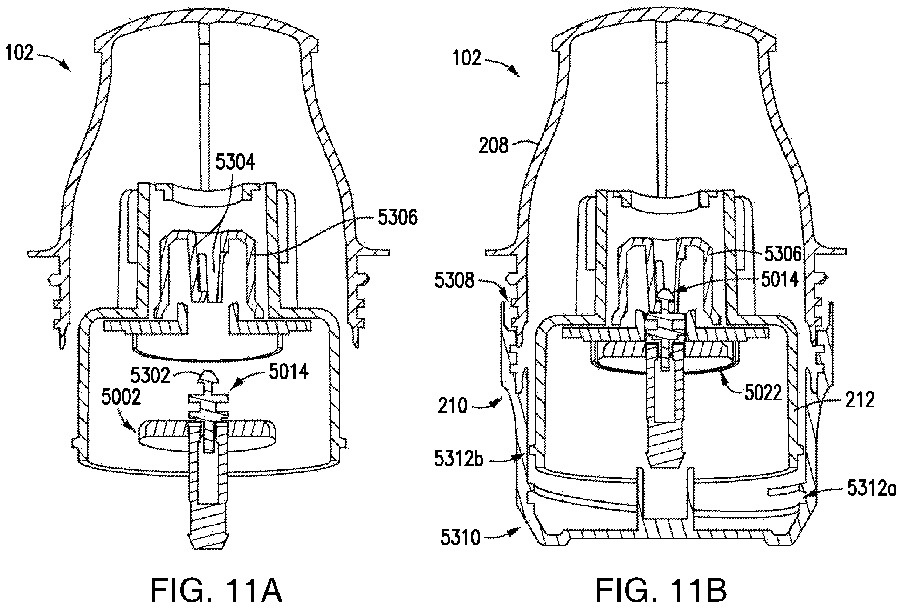

FIGS. 11A-11C are progressive cross-sectional side views showing assembly of the sensor applicator 102 with the sensor control device 5002, according to one or more embodiments. Once the sensor control device 5002 is fully assembled, it may then be loaded into the sensor applicator 102. With reference to FIG. 11A, the sharp hub 5014 may include or otherwise define a hub snap pawl 5302 configured to help couple the sensor control device 5002 to the sensor applicator 102. More specifically, the sensor control device 5002 may be advanced into the interior of the sensor applicator 102 and the hub snap pawl 5302 may be received by corresponding arms 5304 of a sharp carrier 5306 positioned within the sensor applicator 102.

In FIG. 11B, the sensor control device 5002 is shown received by the sharp carrier 5306 and, therefore, secured within the sensor applicator 102. Once the sensor control device 5002 is loaded into the sensor applicator 102, the applicator cap 210 may be coupled to the sensor applicator 102. In some embodiments, the applicator cap 210 and the housing 208 may have opposing, matable sets of threads 5308 that enable the applicator cap 210 to be screwed onto the housing 208 in a clockwise (or counter-clockwise) direction and thereby secure the applicator cap 210 to the sensor applicator 102.

As illustrated, the sheath 212 is also positioned within the sensor applicator 102, and the sensor applicator 102 may include a sheath locking mechanism 5310 configured to ensure that the sheath 212 does not prematurely collapse during a shock event. In the illustrated embodiment, the sheath locking mechanism 5310 may comprise a threaded engagement between the applicator cap 210 and the sheath 212. More specifically, one or more internal threads 53l2a may be defined or otherwise provided on the inner surface of the applicator cap 210, and one or more external threads 53 l2b may be defined or otherwise provided on the sheath 212. The internal and external threads 53 l2a,b may be configured to threadably mate as the applicator cap 210 is threaded to the sensor applicator 102 at the threads 5308. The internal and external threads 53l2a,b may have the same thread pitch as the threads 5308 that enable the applicator cap 210 to be screwed onto the housing 208.

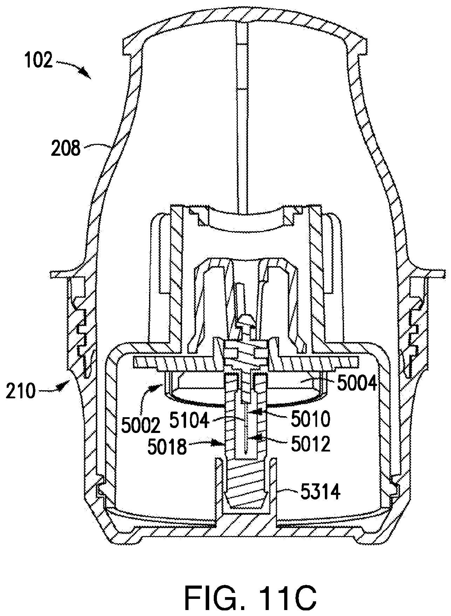

In FIG. 11C, the applicator cap 210 is shown fully threaded (coupled) to the housing 208. As illustrated, the applicator cap 210 may further provide and otherwise define a cap post 5314 centrally located within the interior of the applicator cap 210 and extending proximally from the bottom thereof. The cap post 5314 may be configured to receive at least a portion of the sensor cap 5018 as the applicator cap 210 is screwed onto the housing 208.

With the sensor control device 5002 loaded within the sensor applicator 102 and the applicator cap 210 properly secured, the sensor control device 5002 may then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the tail 5104, and other sensor components, such as membrane coatings that regulate analyte influx.

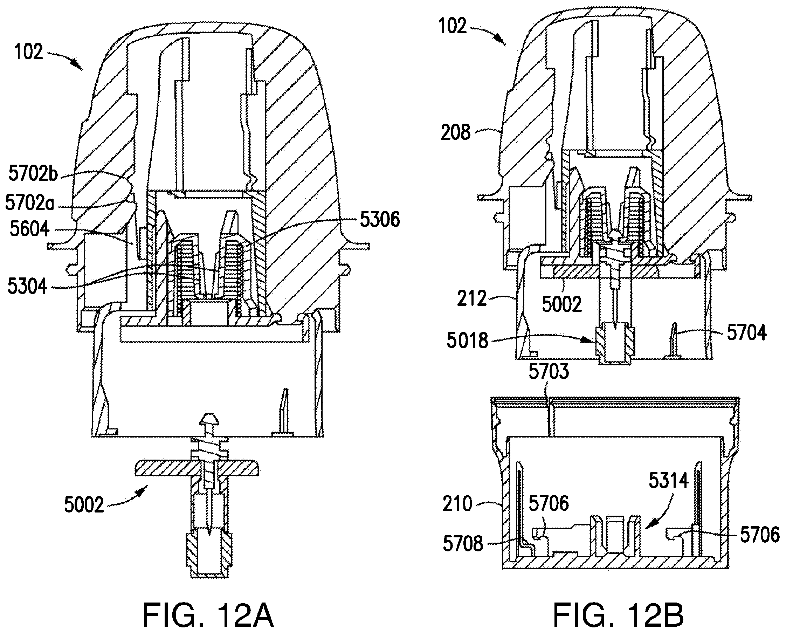

FIGS. 12A-12C are progressive cross-sectional side views showing assembly and disassembly of an alternative embodiment of the sensor applicator 102 with the sensor control device 5002, according to one or more additional embodiments. A fully assembled sensor control device 5002 may be loaded into the sensor applicator 102 by coupling the hub snap pawl 5302 into the arms 5304 of the sharp carrier 5306 positioned within the sensor applicator 102, as generally described above.

In the illustrated embodiment, the sheath arms 5604 of the sheath 212 may be configured to interact with a first detent 5702a and a second detent 5702b defined within the interior of the housing 208. The first detent 5702a may alternately be referred to a "locking" detent, and the second detent 5702b may alternately be referred to as a "firing" detent. When the sensor control device 5002 is initially installed in the sensor applicator 102, the sheath arms 5604 may be received within the first detent 5702a. As discussed below, the sheath 212 may be actuated to move the sheath arms 5604 to the second detent 5702b, which places the sensor applicator 102 in firing position.

In FIG. 12B, the applicator cap 210 is aligned with the housing 208 and advanced toward the housing 208 so that the sheath 212 is received within the applicator cap 210. Instead of rotating the applicator cap 210 relative to the housing 208, the threads of the applicator cap 210 may be snapped onto the corresponding threads of the housing 208 to couple the applicator cap 210 to the housing 208. Axial cuts or slots 5703 (one shown) defined in the applicator cap 210 may allow portions of the applicator cap 210 near its threading to flex outward to be snapped into engagement with the threading of the housing 208. As the applicator cap 210 is snapped to the housing 208, the sensor cap 5018 may correspondingly be snapped into the cap post 5314.

Similar to the embodiment of FIGS. 11A-11C, the sensor applicator 102 may include a sheath locking mechanism configured to ensure that the sheath 212 does not prematurely collapse during a shock event. In the illustrated embodiment, the sheath locking mechanism includes one or more ribs 5704 (one shown) defined near the base of the sheath 212 and configured to interact with one or more ribs 5706 (two shown) and a shoulder 5708 defined near the base of the applicator cap 210. The ribs 5704 may be configured to inter-lock between the ribs 5706 and the shoulder 5708 while attaching the applicator cap 210 to the housing 208. More specifically, once the applicator cap 210 is snapped onto the housing 208, the applicator cap 210 may be rotated (e.g., clockwise), which locates the ribs 5704 of the sheath 212 between the ribs 5706 and the shoulder 5708 of the applicator cap 210 and thereby "locks" the applicator cap 210 in place until the user reverse rotates the applicator cap 210 to remove the applicator cap 210 for use. Engagement of the ribs 5704 between the ribs 5706 and the shoulder 5708 of the applicator cap 210 may also prevent the sheath 212 from collapsing prematurely.

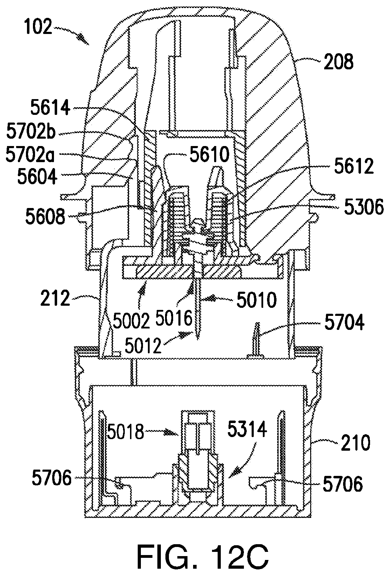

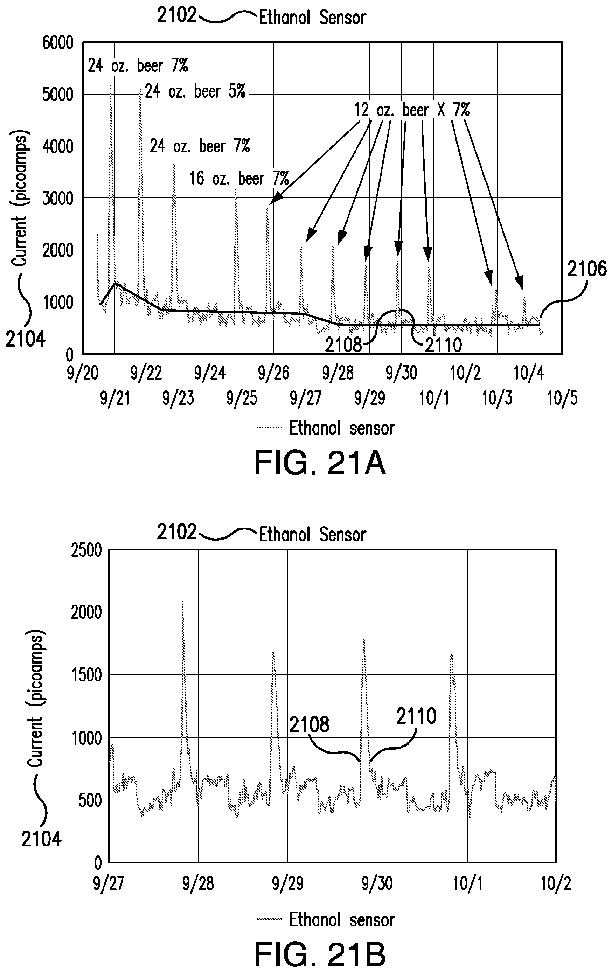

In FIG. 12C, the applicator cap 210 is removed from the housing 208. As with the embodiment of FIGS. 21A-21C, the applicator cap 210 can be removed by reverse rotating the applicator cap 210, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 210 is unscrewed from the housing 208, the ribs 5704 defined on the sheath 212 may slidingly engage the tops of the ribs 5706 defined on the applicator cap 210. The tops of the ribs 5706 may provide corresponding ramped surfaces that result in an upward displacement of the sheath 212 as the applicator cap 210 is rotated, and moving the sheath 212 upward causes the sheath arms 5604 to flex out of engagement with the first detent 5702a to be received within the second detent 5702b. As the sheath 212 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 208, the mating member 5016 may correspondingly retract until it becomes flush, substantially flush, or sub-flush with the bottom of the sensor control device 5002. At this point, the sensor applicator 102 in firing position. Accordingly, in this embodiment, removing the applicator cap 210 correspondingly causes the mating member 5016 to retract.

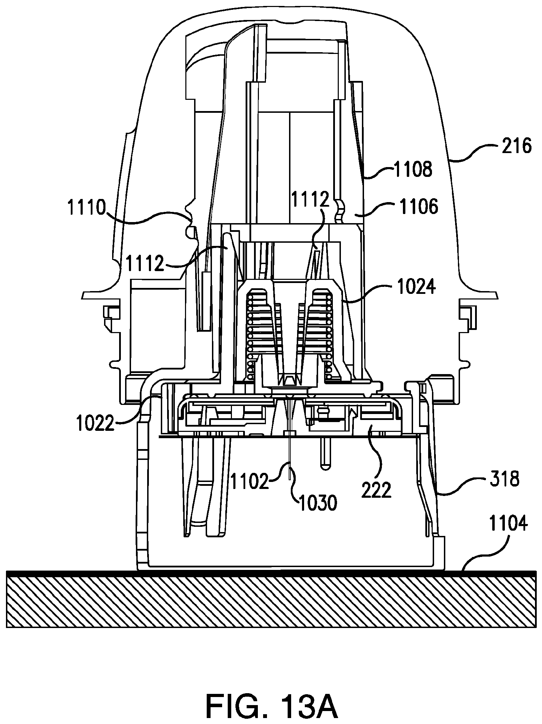

FIGS. 13A-13F illustrate example details of embodiments of the internal device mechanics of "firing" the applicator 216 to apply sensor control device 222 to a user and including retracting sharp 1030 safely back into used applicator 216. All together, these drawings represent an example sequence of driving sharp 1030 (supporting a sensor coupled to sensor control device 222) into the skin of a user, withdrawing the sharp while leaving the sensor behind in operative contact with interstitial fluid of the user, and adhering the sensor control device to the skin of the user with an adhesive. Modification of such activity for use with the alternative applicator assembly embodiments and components can be appreciated in reference to the same by those with skill in the art. Moreover, applicator 216 may be a sensor applicator having one-piece architecture or a two-piece architecture as disclosed herein.

Turning now to FIG. 13A, a sensor 1102 is supported within sharp 1030, just above the skin 1104 of the user. Rails 1106 (optionally three of them) of an upper guide section 1108 may be provided to control applicator 216 motion relative to sheath 318. The sheath 318 is held by detent features 1110 within the applicator 216 such that appropriate downward force along the longitudinal axis of the applicator 216 will cause the resistance provided by the detent features 1110 to be overcome so that sharp 1030 and sensor control device 222 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 222.

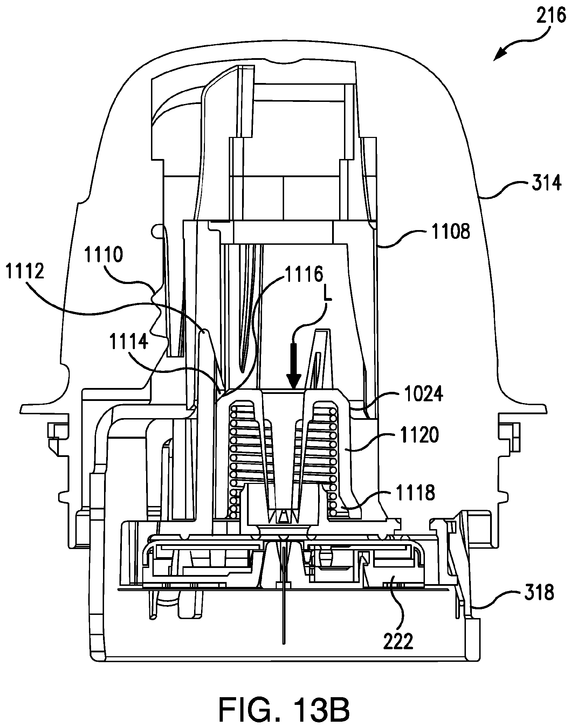

In FIG. 13B, user force is applied to overcome or override detent features 1110 and sheath 318 collapses into housing 314 driving the sensor control device 222 (with associated parts) to translate down as indicated by the arrow L along the longitudinal axis. An inner diameter of the upper guide section 1108 of the sheath 318 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized. According to embodiments, rather than employing user force to drive the sensor control device 222 to translate down as indicated by the arrow L along the longitudinal axis, housing 314 can include a button (for example, not limitation, a push button) which activates a drive spring (for example, not limitation, a coil spring) to drive the sensor control device 222.

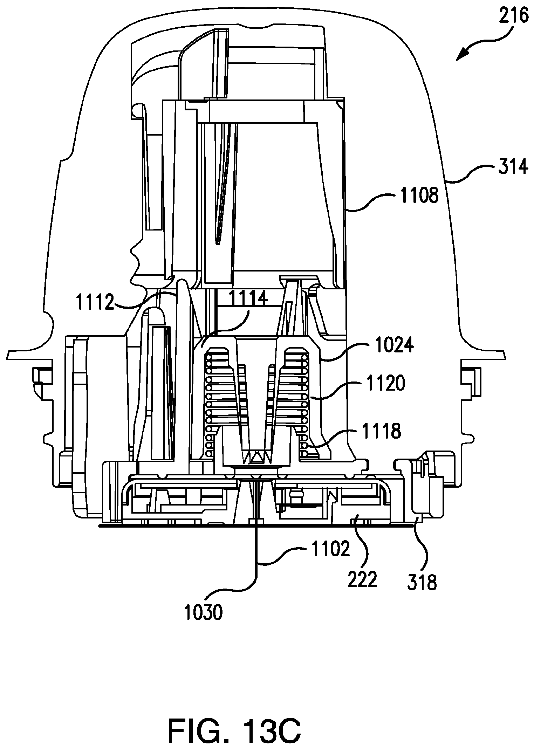

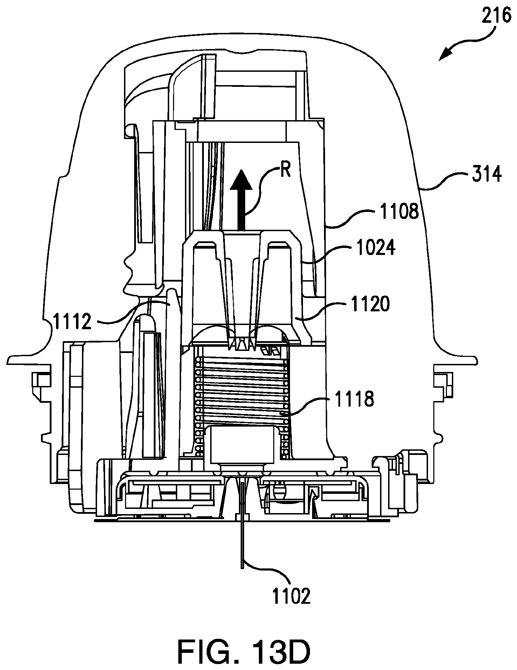

In FIG. 13C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 13D.

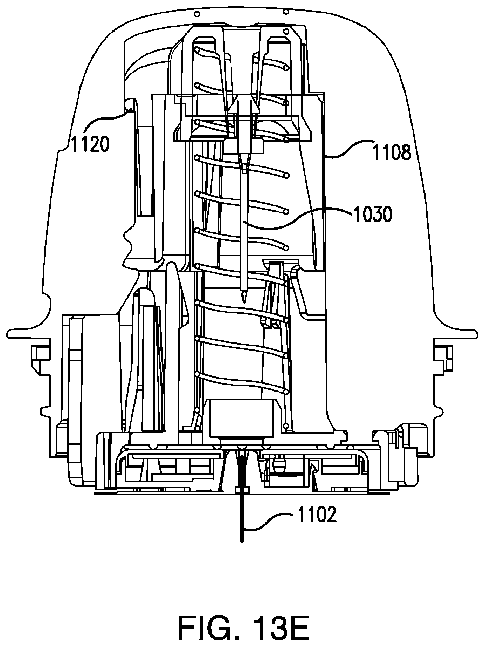

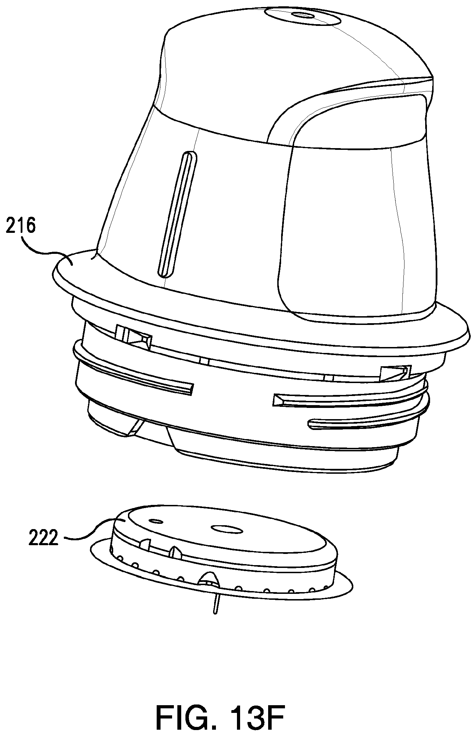

With the sharp 1030 fully retracted as shown in FIG. 13E, the upper guide section 1108 of the sheath 318 is set with a final locking feature 1120. As shown in FIG. 13F, the spent applicator assembly 216 is removed from the insertion site, leaving behind the sensor control device 222, and with the sharp 1030 secured safely inside the applicator assembly 216. The spent applicator assembly 216 is now ready for disposal.

Operation of the applicator 216 when applying the sensor control device 222 is designed to provide the user with a sensation that both the insertion and retraction of the sharp 1030 is performed automatically by the internal mechanisms of the applicator 216. In other words, the present invention avoids the user experiencing the sensation that he is manually driving the sharp 1030 into his skin. Thus, once the user applies sufficient force to overcome the resistance from the detent features of the applicator 216, the resulting actions of the applicator 216 are perceived to be an automated response to the applicator being "triggered." The user does not perceive that he is supplying additional force to drive the sharp 1030 to pierce his skin despite that all the driving force is provided by the user and no additional biasing/driving means are used to insert the sharp 1030. As detailed above in FIG. 13C, the retraction of the sharp 1030 is automated by the coil return spring 1118 of the applicator 216.

With respect to any of the applicator embodiments described herein, as well as any of the components thereof, including but not limited to the sharp, sharp module and sensor module embodiments, those of skill in the art will understand that said embodiments can be dimensioned and configured for use with sensors configured to sense an analyte level in a bodily fluid in the epidermis, dermis, or subcutaneous tissue of a subject. In some embodiments, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator embodiments, those of skill in the art will appreciate that certain embodiments of sharps can be dimensioned and configured to be positioned at a different end-depth in the subject's body relative to the final end-depth of the analyte sensor. In some embodiments, for example, a sharp can be positioned at a first end-depth in the subject's epidermis prior to retraction, while a distal portion of an analyte sensor can be positioned at a second end-depth in the subject's dermis. In other embodiments, a sharp can be positioned at a first end-depth in the subject's dermis prior to retraction, while a distal portion of an analyte sensor can be positioned at a second end-depth in the subject's subcutaneous tissue. In still other embodiments, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator embodiments described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator embodiments, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator embodiments, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019.

Further details regarding embodiments of applicators, their components, and variants thereof, are described in U.S. Patent Publication Nos. 2013/0150691, 2016/0331283, and 2018/0235520.

Further details regarding embodiments of sharp modules, sharps, their components, and variants thereof, are described in U.S. Patent Publication No. 2014/0171771.

Biochemical sensors can be described by one or more sensing characteristics. A common sensing characteristic is referred to as the biochemical sensor's sensitivity, which is a measure of the sensor's responsiveness to the concentration of the chemical or composition it is designed to detect. For electrochemical sensors, this response can be in the form of an electrical current (amperometric) or electrical charge (coulometric). For other types of sensors, the response can be in a different form, such as a photonic intensity (e.g., optical light). The sensitivity of a biochemical analyte sensor can vary depending on a number of factors, including whether the sensor is in an in vitro state or an in vivo state.

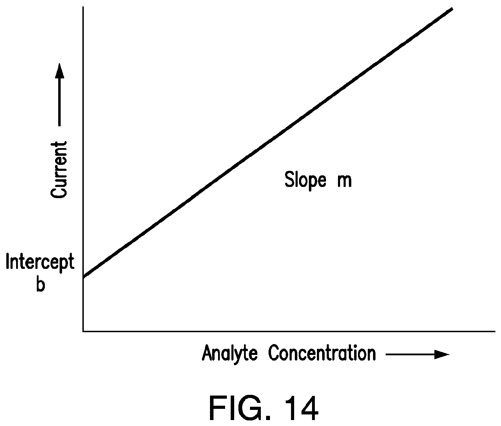

FIG. 14 is a graph depicting the in vitro sensitivity of an amperometric analyte sensor. The in vitro sensitivity can be obtained by in vitro testing the sensor at various analyte concentrations and then performing a regression (e.g., linear or non-linear) or other curve fitting on the resulting data. In this example, the analyte sensor's sensitivity is linear, or substantially linear, and can be modeled according to the equation $y = mx + b$, where $y$ is the sensor's electrical output current, $x$ is the analyte level (or concentration), $m$ is the slope of the sensitivity and $b$ is the intercept of the sensitivity, where the intercept generally corresponds to a background signal (e.g., noise). For sensors with a linear or substantially linear response, the analyte level that corresponds to a given current can be determined from the slope and intercept of the sensitivity. Sensors with a non-linear sensitivity require additional information to determine the analyte level resulting from the sensor's output current, and those of ordinary skill in the art are familiar with manners by which to model non-linear sensitivities. In certain embodiments of in vivo sensors, the in vitro sensitivity can be the same as the in vivo sensitivity, but in other embodiments a transfer (or conversion) function is used to translate the in vitro sensitivity into the in vivo sensitivity that is applicable to the sensor's intended in vivo use.

Calibration is a technique for improving or maintaining accuracy by adjusting a sensor's measured output to reduce the differences with the sensor's expected output. One or more parameters that describe the sensor's sensing characteristics, like its sensitivity, are established for use in the calibration adjustment.

Certain in vivo analyte monitoring systems require calibration to occur after implantation of the sensor into the user or patient, either by user interaction or by the system itself in an automated fashion. For example, when user interaction is required, the user performs an in vitro measurement (e.g., a blood glucose (BG) measurement using a finger stick and an in vitro test strip) and enters this into the system, while the analyte sensor is implanted. The system then compares the in vitro measurement with the in vivo signal and, using the differential, determines an estimate of the sensor's in vivo sensitivity. The in vivo sensitivity can then be used in an algorithmic process to transform the data collected with the sensor to a value that indicates the user's analyte level. This and other processes that require user action to perform calibration are referred to as "user calibration." Systems can require user calibration due to instability of the sensor's sensitivity, such that the sensitivity drifts or changes over time. Thus, multiple user calibrations (e.g., according to a periodic (e.g., daily) schedule, variable schedule, or on an as-needed basis) can be required to maintain accuracy. While the embodiments described herein can incorporate a degree of user calibration for a particular implementation, generally this is not preferred as it requires the user to perform a painful or otherwise burdensome BG measurement, and can introduce user error.

Some in vivo analyte monitoring systems can regularly adjust the calibration parameters through the use of automated measurements of characteristics of the sensor made by the system itself (e.g., processing circuitry executing software). The repeated adjustment of the sensor's sensitivity based on a variable measured by the system (and not the user) is referred to generally as "system" (or automated) calibration, and can be performed with user calibration, such as an early BG measurement, or without user calibration. Like the case with repeated user calibrations, repeated system calibrations are typically necessitated by drift in the sensor's sensitivity over time. Thus, while the embodiments described herein can be used with a degree of automated system calibration, preferably the sensor's sensitivity is relatively stable over time such that post-implantation calibration is not required.

Some In vivo analyte monitoring systems operate with a sensor that is factory calibrated. Factory calibration refers to the determination or estimation of the one or more calibration parameters prior to distribution to the user or healthcare professional (HCP). The calibration parameter can be determined by the sensor manufacturer (or the manufacturer of the other components of the sensor control device if the two entities are different). Many in vivo sensor manufacturing processes fabricate the sensors in groups or batches referred to as production lots, manufacturing stage lots, or simply lots. A single lot can include thousands of sensors.

Sensors can include a calibration code or parameter which can be derived or determined during one or more sensor manufacturing processes and coded or programmed, as part of the manufacturing process, in the data processing device of the analyte monitoring system or provided on the sensor itself, for example, as a bar code, a laser tag, an RFID tag, or other machine readable information provided on the sensor. User calibration during in vivo use of the sensor can be obviated, or the frequency of in vivo calibrations during sensor wear can be reduced if the code is provided to a receiver (or other data processing device). In embodiments where the calibration code or parameter is provided on the sensor itself, prior to or at the start of the sensor use, the calibration code or parameter can be automatically transmitted or provided to the data processing device in the analyte monitoring system.

Some In vivo analyte monitoring system operate with a sensor that can be one or more of factory calibrated, system calibrated, and/or user calibrated. For example, the sensor can be provided with a calibration code or parameter which can allow for factory calibration. If the information is provided to a receiver (for example, entered by a user), the sensor can operate as a factory calibrated sensor. If the information is not provided to a receiver, the sensor can operate as a user calibrated sensor and/or a system calibrated sensor.

In a further aspect, programming or executable instructions can be provided or stored in the data processing device of the analyte monitoring system, and/or the receiver/controller unit, to provide a time varying adjustment algorithm to the in vivo sensor during use. For example, based on a retrospective statistical analysis of analyte sensors used in vivo and the corresponding glucose level feedback, a predetermined or analytical curve or a database can be generated which is time based, and configured to provide additional adjustment to the one or more in vivo sensor parameters to compensate for potential sensor drift in stability profile, or other factors.

In accordance with the disclosed subject matter, the analyte monitoring system can be configured to compensate or adjust for the sensor sensitivity based on a sensor drift profile. A time varying parameter $\beta(t)$ can be defined or determined based on analysis of sensor behavior during in vivo use, and a time varying drift profile can be determined. In certain aspects, the compensation or adjustment to the sensor sensitivity can be programmed in the receiver unit, the controller or data processor of the analyte monitoring system such that the compensation or the adjustment or both can be performed automatically and/or iteratively when sensor data is received from the analyte sensor. In accordance with the disclosed subject matter, the adjustment or compensation algorithm can be initiated or executed by the user (rather than self-initiating or executing) such that the adjustment or the compensation to the analyte sensor sensitivity profile is performed or executed upon user initiation or activation of the corresponding function or routine, or upon the user entering the sensor calibration code.

In accordance with the disclosed subject matter, each sensor in the sensor lot (in some instances not including sample sensors used for in vitro testing) can be examined non-destructively to determine or measure its characteristics such as membrane thickness at one or more points of the sensor, and other characteristics including physical characteristics such as the surface area/volume of the active area can be measured or determined. Such measurement or determination can be performed in an automated manner using, for example, optical scanners or other suitable measurement devices or systems, and the determined sensor characteristics for each sensor in the sensor lot is compared to the corresponding mean values based on the sample sensors for possible correction of the calibration parameter or code assigned to each sensor. For example, for a calibration parameter defined as the sensor sensitivity, the sensitivity is approximately inversely proportional to the membrane thickness, such that, for example, a sensor having a measured membrane thickness of approximately 4% greater than the mean membrane thickness for the sampled sensors from the same sensor lot as the sensor, the sensitivity assigned to that sensor in one embodiment is the mean sensitivity determined from the sampled sensors divided by 1.04. Likewise, since the sensitivity is approximately proportional to active area of the sensor, a sensor having measured active area of approximately 3% lower than the mean active area for the sampled sensors from the same sensor lot, the sensitivity assigned to that sensor is the mean sensitivity multiplied by 0.97. The assigned sensitivity can be determined from the mean sensitivity from the sampled sensors, by multiple successive adjustments for each examination or measurement of the sensor. In certain embodiments, examination or measurement of each sensor can additionally include measurement of membrane consistency or texture in addition to the membrane thickness and/or surface are or volume of the active sensing area.

Additional information regarding sensor calibration is provided in U.S. Publication No. 2010/00230285 and U.S. Publication No. 2019/0274598.

The storage memory 5030 of the sensor 110 can include the software blocks related to communication protocols of the communication module. For example, the storage memory 5030 can include a BLE services software block with functions to provide interfaces to make the BLE module 5041 available to the computing hardware of the sensor 110. These software functions can include a BLE logical interface and interface parser. BLE services offered by the communication module 5040 can include the generic access profile service, the generic attribute service, generic access service, device information service, data transmission services, and security services. The data transmission service can be a primary service used for transmitting data such as sensor control data, sensor status data, analyte measurement data (historical and current), and event log data. The sensor status data can include error data, current time active, and software state. The analyte measurement data can include information such as current and historical raw measurement values, current and historical values after processing using an appropriate algorithm or model, projections and trends of measurement levels, comparisons of other values to patient-specific averages, calls to action as determined by the algorithms or models and other similar types of data.

According to aspects of the disclosed subject matter, and as embodied herein, a sensor 110 can be configured to communicate with multiple devices concurrently by adapting the features of a communication protocol or medium supported by the hardware and radios of the sensor 110. As an example, the BLE module 5041 of the communication module 5040 can be provided with software or firmware to enable multiple concurrent connections between the sensor 110 as a central device and the other devices as peripheral devices, or as a peripheral device where another device is a central device.

Connections, and ensuing communication sessions, between two devices using a communication protocol such as BLE can be characterized by a similar physical channel operated between the two devices (e.g., a sensor 110 and data receiving device 120). The physical channel can include a single channel or a series of channels, including for example and without limitation using an agreed upon series of channels determined by a common clock and channel- or frequency-hopping sequence. Communication sessions can use a similar amount of the available communication spectrum, and multiple such communication sessions can exist in proximity. In certain embodiment, each collection of devices in a communication session uses a different physical channel or series of channels, to manage interference of devices in the same proximity.

For purpose of illustration and not limitation, reference is made to an exemplary embodiment of a procedure for a sensor-receiver connection for use with the disclosed subject matter. First, the sensor 110 repeatedly advertises its connection information to its environment in a search for a data receiving device 120. The sensor 110 can repeat advertising on a regular basis until a connection established. The data receiving device 120 detects the advertising packet and scans and filters for the sensor 120 to connect to through the data provided in the advertising packet. Next, data receiving device 120 sends a scan request command and the sensor 110 responds with a scan response packet providing additional details. Then, the data receiving device 120 sends a connection request using the Bluetooth device address associated with the data receiving device 120. The data receiving device 120 can also continuously request to establish a connection to a sensor 110 with a specific Bluetooth device address. Then, the devices establish an initial connection allowing them to begin to exchange data. The devices begin a process to initialize data exchange services and perform a mutual authentication procedure.

During a first connection between the sensor 110 and data receiving device 120, the data receiving device 120 can initialize a service, characteristic, and attribute discovery procedure. The data receiving device 120 can evaluate these features of the sensor 110 and store them for use during subsequent connections. Next, the devices enable a notification for a customized security service used for mutual authentication of the sensor 110 and data receiving device 120. The mutual authentication procedure can be automated and require no user interaction. Following the successful completion of the mutual authentication procedure, the sensor 110 sends a connection parameter update to request the data receiving device 120 to use connection parameter settings preferred by the sensor 110 and configured to maximum longevity.

The data receiving device 120 then performs sensor control procedures to backfill historical data, current data, event log, and factory data. As an example, for each type of data, the data receiving device 120 sends a request to initiate a backfill process. The request can specify a range of records defined based on, for example, the measurement value, timestamp, or similar, as appropriate. The sensor 110 responds with requested data until all previously unsent data in the memory of the sensor 110 is delivered to the data receiving device 120. The sensor 110 can respond to a backfill request from the data receiving device 120 that all data has already been sent. Once backfill is completed, the data receiving device 120 can notify sensor 110 that it is ready to receive regular measurement readings. The sensor 110 can send readings across multiple notifications result on a repeating basis. As embodied herein, the multiple notifications can be redundant notifications to ensure that data is transmitted correctly. Alternatively, multiple notifications can make up a single payload.

For purpose of illustration and not limitation, reference is made to an exemplary embodiment of a procedure to send a shutdown command to the sensor 110. The shutdown operation is executed if the sensor 110 is in, for example, an error state, insertion failed state, or sensor expired state. If the sensor 110 is not in those states, the sensor 110 can log the command and execute the shutdown when sensor 110 transitions into the error state or sensor expired state. The data receiving device 120 sends a properly formatted shutdown command to the sensor 110. If the sensor 110 is actively processing another command, the sensor 110 will respond with a standard error response indicating that the sensor 110 is busy. Otherwise, the sensor 110 sends a response as the command is received. Additionally, the sensor 110 sends a success notification through the sensor control characteristic to acknowledge the sensor 110 has received the command. The sensor 110 registers the shutdown command. At the next appropriate opportunity (e.g., depending on the current sensor state, as described herein), the sensor 110 will shut down.

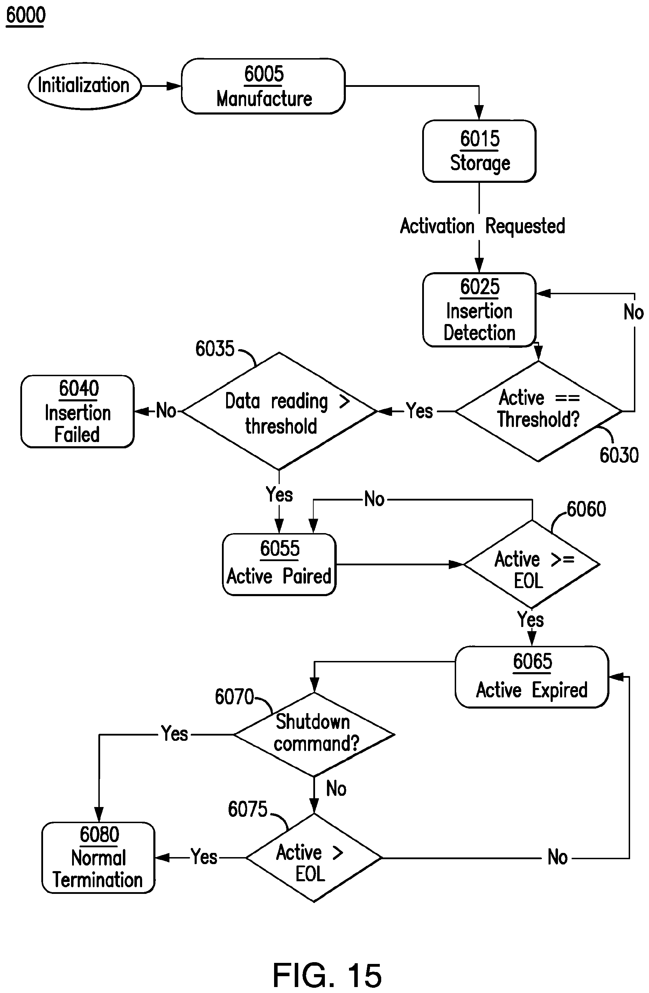

For purpose of illustration and not limitation, reference is made to the exemplary embodiment of a high-level depiction of a state machine representation 6000 of the actions that can be taken by the sensor 110 as shown in FIG. 15. After initialization, the sensor enters state 6005, which relates to the manufacture of the sensor 110. In the manufacture state 6005 the sensor 110 can be configured for operation, for example, the storage memory 5030 can be written. At various times while in state 6005, the sensor 110 checks for a received command to go to the storage state 6015. Upon entry to the storage state 6015, the sensor performs a software integrity check. While in the storage state 6015, the sensor can also receive an activation request command before advancing to the insertion detection state 6025.

Upon entry to state 6025, the sensor 110 can store information relating to devices authenticated to communicate with the sensor as set during activation or initialize algorithms related to conducting and interpreting measurements from the sensing hardware 5060. The sensor 110 can also initialize a lifecycle timer, responsible for maintaining an active count of the time of operation of the sensor 110 and begin communication with authenticated devices to transmit recorded data. While in the insertion detection state 6025, the sensor can enter state 6030, where the sensor 110 checks whether the time of operation is equal to a predetermined threshold. This time of operation threshold can correspond to a timeout function for determining whether an insertion has been successful. If the time of operation has reached the threshold, the sensor 110 advances to state 6035, in which the sensor 110 checks whether the average data reading is greater than a threshold amount corresponding to an expected data reading volume for triggering detection of a successful insertion. If the data reading volume is lower than the threshold while in state 6035, the sensor advances to state 6040, corresponding to a failed insertion. If the data reading volume satisfies the threshold, the sensor advances to the active paired state 6055.

The active paired state 6055 of the sensor 110 reflects the state while the sensor 110 is operating as normal by recording measurements, processing the measurements, and reporting them as appropriate. While in the active paired state 6055, the sensor 110 sends measurement results or attempts to establish a connection with a receiving device 120. The sensor 110 also increments the time of operation. Once the sensor 110 reaches a predetermined threshold time of operation (e.g., once the time of operation reaches a predetermined threshold), the sensor 110 transitions to the active expired state 6065. The active expired state 6065 of the sensor 110 reflects the state while the sensor 110 has operated for its maximum predetermined amount of time.

While in the active expired state 6065, the sensor 110 can generally perform operations relating to winding down operation and ensuring that the collected measurements have been securely transmitted to receiving devices as needed. For example, while in the active expired state 6065, the sensor 110 can transmit collected data and, if no connection is available, can increase efforts to discover authenticated devices nearby and establish and connection therewith. While in the active expired state 6065, the sensor 110 can receive a shutdown command at state 6070. If no shutdown command is received, the sensor 110 can also, at state 6075, check if the time of operation has exceeded a final operation threshold. The final operation threshold can be based on the battery life of the sensor 110. The normal termination state 6080 corresponds to the final operations of the sensor 110 and ultimately shutting down the sensor 110.

Before a sensor is activated, the ASIC 5000 resides in a low power storage mode state. The activation process can begin, for example, when an incoming RF field (e.g., NFC field) drives the voltage of the power supply to the ASIC 5000 above a reset threshold, which causes the sensor 110 to enter a wake-up state. While in the wake-up state, the ASIC 5000 enters an activation sequence state. The ASIC 5000 then wakes the communication module 5040. The communication module 5040 is initialized, triggering a power on self-test. The power on self-test can include the ASIC 5000 communicating with the communication module 5040 using a prescribed sequence of reading and writing data to verify the memory and one-time programmable memory are not corrupted.

When the ASIC 5000 enters the measurement mode for the first time, an insertion detection sequence is performed to verify that the sensor 110 has been properly installed onto the patient's body before a proper measurement can take place. First, the sensor 110 interprets a command to activate the measurement configuration process, causing the ASIC 5000 to enter measurement command mode. The sensor 110 then temporarily enters the measurement lifecycle state to run a number of consecutive measurements to test whether the insertion has been successful. The communication module 5040 or ASIC 5000 evaluates the measurement results to determine insertion success. When insertion is deemed successful, the sensor 110 enters a measurement state, in which the sensor 110 begins taking regular measurements using sensing hardware 5060. If the sensor 110 determines that the insertion was not successful, sensor 110 is triggered into an insertion failure mode, in which the ASIC 5000 is commanded back to storage mode while the communication module 5040 disables itself.

FIG. 1A further illustrates an example operating environment for providing over-the-air ("OTA") updates for use with the techniques described herein. An operator of the analyte monitoring system 100 can bundle updates for the data receiving device 120 or sensor 110 into updates for an application executing on the multi-purpose data receiving device 130. Using available communication channels between the data receiving device 120, the multi-purpose data receiving device 130, and the sensor 110, the multi-purpose data receiving device 130 can receive regular updates for the data receiving device 120 or sensor 110 and initiate installation of the updates on the data receiving device 120 or sensor 110. The multi-purpose data receiving device 130 acts as an installation or update platform for the data receiving device 120 or sensor 110 because the application that enables the multi-purpose data receiving device 130 to communicate with an analyte sensor 110, data receiving device 120 and/or remote application server 150 can update software or firmware on a data receiving device 120 or sensor 110 without wide-area networking capabilities.

As embodied herein, a remote application server 150 operated by the manufacturer of the analyte sensor 110 and/or the operator of the analyte monitoring system 100 can provide software and firmware updates to the devices of the analyte monitoring system 100. In particular embodiments, the remote application server 150 can provides the updated software and firmware to a user device 140 or directly to a multi-purpose data receiving device. As embodied herein, the remote application server 150 can also provide application software updates to an application storefront server 160 using interfaces provided by the application storefront. The multi-purpose data receiving device 130 can contact the application storefront server 160 periodically to download and install the updates.

After the multi-purpose data receiving device 130 downloads an application update including a firmware or software update for a data receiving device 120 or sensor 110, the data receiving device 120 or sensor 110 and multi-purpose data receiving device 130 establish a connection. The multi-purpose data receiving device 130 determines that a firmware or software update is available for the data receiving device 120 or sensor 110. The multi-purpose data receiving device 130 can prepare the software or firmware update for delivery to the data receiving device 120 or sensor 110. As an example, the multi-purpose data receiving device 130 can compress or segment the data associated with the software or firmware update, can encrypt or decrypt the firmware or software update, or can perform an integrity check of the firmware or software update. The multi-purpose data receiving device 130 sends the data for the firmware or software update to the data receiving device 120 or sensor 110. The multi-purpose data receiving device 130 can also send a command to the data receiving device 120 or sensor 110 to initiate the update. Additionally or alternatively, the multi-purpose data receiving device 130 can provide a notification to the user of the multi-purpose data receiving device 130 and include instructions for facilitating the update, such as instructions to keep the data receiving device 120 and the multi-purpose data receiving device 130 connected to a power source and in close proximity until the update is complete.

The data receiving device 120 or sensor 110 receives the data for the update and the command to initiate the update from the multi-purpose data receiving device 130. The data receiving device 120 can then install the firmware or software update. To install the update, the data receiving device 120 or sensor 110 can place or restart itself in a so-called "safe" mode with limited operational capabilities. Once the update is completed, the data receiving device 120 or sensor 110 re-enters or resets into a standard operational mode. The data receiving device 120 or sensor 110 can perform one or more self-tests to determine that the firmware or software update was installed successfully. The multi-purpose data receiving device 130 can receive the notification of the successful update. The multi-purpose data receiving device 130 can then report a confirmation of the successful update to the remote application server 150.

In particular embodiments, the storage memory 5030 of the sensor 110 includes one-time programmable (OTP) memory. The term OTP memory can refer to memory that includes access restrictions and security to facilitate writing to particular addresses or segments in the memory a predetermined number of times. The memory 5030 can be prearranged into multiple pre-allocated memory blocks or containers. The containers are pre-allocated into a fixed size. If storage memory 5030 is one-time programming memory, the containers can be considered to be in a non-programmable state. Additional containers which have not yet been written to can be placed into a programmable or writable state. Containerizing the storage memory 5030 in this fashion can improve the transportability of code and data to be written to the storage memory 5030. Updating the software of a device (e.g., the sensor device described herein) stored in an OTP memory can be performed by superseding only the code in a particular previously-written container or containers with updated code written to a new container or containers, rather than replacing the entire code in the memory. In a second embodiment, the memory is not prearranged. Instead, the space allocated for data is dynamically allocated or determined as needed. Incremental updates can be issued, as containers of varying sizes can be defined where updates are anticipated.

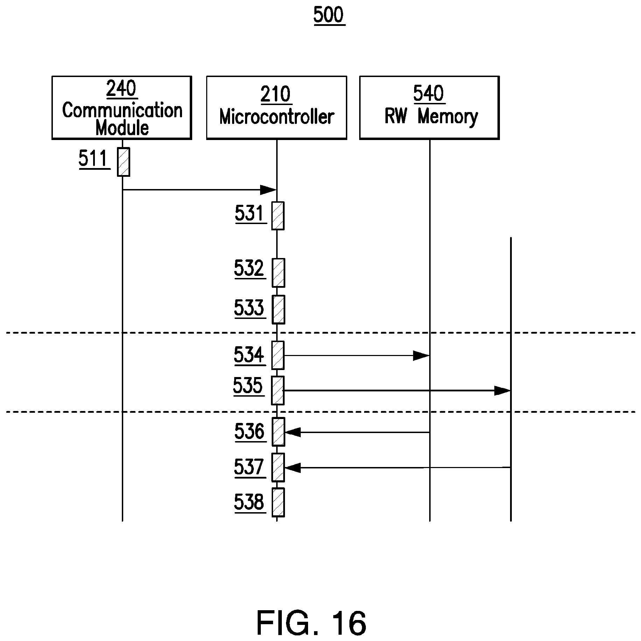

FIG. 16 is a diagram illustrating an example operational and data flow for over-the-air (OTA) programming of a storage memory 5030 in a sensor device 100 as well as use of the memory after the OTA programming in execution of processes by the sensor device 110 according to the disclosed subject matter. In the example OTA programming 500 illustrated in FIG. 5, a request is sent from an external device (e.g., the data receiving device 130) to initiate OTA programming (or re-programming). At 511, a communication module 5040 of a sensor device 110 receives an OTA programming command. The communication module 5040 sends the OTA programming command to the microcontroller 5010 of the sensor device 110.

At 531, after receiving the OTA programming command, the microcontroller 5010 validates the OTA programming command. The microcontroller 5010 can determine, for example, whether the OTA programming command is signed with an appropriate digital signature token. Upon determining that the OTA programming command is valid, the microcontroller 5010 can set the sensor device into an OTA programming mode. At 532, the microcontroller 5010 can validate the OTA programming data. At 533, The microcontroller 5010 can reset the sensor device 110 to re-initialize the sensor device 110 in a programming state. Once the sensor device 110 has transitioned into the OTA programming state, the microcontroller 5010 can begin to write data to the rewriteable memory 540 (e.g., memory 5020) of the sensor device at 534 and write data to the OTP memory 550 of the sensor device at 535 (e.g., storage memory 5030). The data written by the microcontroller 5010 can be based on the validated OTA programming data. The microcontroller 5010 can write data to cause one or more programming blocks or regions of the OTP memory 550 to be marked invalid or inaccessible. The data written to the free or unused portion of the OTP memory can be used to replace invalidated or inaccessible programming blocks of the OTP memory 550. After the microcontroller 5010 writes the data to the respective memories at 534 and 535, the microcontroller 5010 can perform one or more software integrity checks to ensure that errors were not introduced into the programming blocks during the writing process. Once the microcontroller 5010 is able to determine that the data has been written without errors, the microcontroller 5010 can resume standard operations of the sensor device.

In execution mode, at 536, the microcontroller 5010 can retrieve a programming manifest or profile from the rewriteable memory 540. The programming manifest or profile can include a listing of the valid software programming blocks and can include a guide to program execution for the sensor 110. By following the programming manifest or profile, the microcontroller 5010 can determine which memory blocks of the OTP memory 550 are appropriate to execute and avoid execution of out-of-date or invalidated programming blocks or reference to out-of-date data. At 537, the microcontroller 5010 can selectively retrieve memory blocks from the OTP memory 550. At 538, the microcontroller 5010 can use the retrieved memory blocks, by executing programming code stored or using variable stored in the memory.

As embodied herein a first layer of security for communications between the analyte sensor 110 and other devices can be established based on security protocols specified by and integrated in the communication protocols used for the communication. Another layer of security can be based on communication protocols that necessitate close proximity of communicating devices. Furthermore certain packets and/or certain data included within packets can be encrypted while other packets and/or data within packets is otherwise encrypted or not encrypted. Additionally or alternatively, application layer encryption can be used with one or more block ciphers or stream ciphers to establish mutual authentication and communication encryption with other devices in the analyte monitoring system 100.

The ASIC 5000 of the analyte sensor 110 can be configured to dynamically generate authentication and encryption keys using data retained within the storage memory 5030. The storage memory 5030 can also be pre-programmed with a set of valid authentication and encryption keys to use with particular classes of devices. The ASIC 5000 can be further configured to perform authentication procedures with other devices using received data and apply the generated key to sensitive data prior to transmitting the sensitive data. The generated key can be unique to the analyte sensor 110, unique to a pair of devices, unique to a communication session between an analyte sensor 110 and other device, unique to a message sent during a communication session, or unique to a block of data contained within a message.

Both the sensor 110 and a data receiving device 120 can ensure the authorization of the other party in a communication session to, for example, issue a command or receive data. In particular embodiments, identity authentication can be performed through two features. First, the party asserting its identity provides a validated certificate signed by the manufacturer of the device or the operator of the analyte monitoring system 100. Second, authentication can be enforced through the use of public keys and private keys, and shared secrets derived therefrom, established by the devices of the analyte monitoring system 100 or established by the operator of the analyte monitoring system 100. To confirm the identity of the other party, the party can provide proof that the party has control of its private key.

The manufacturer of the analyte sensor 110, data receiving device 120, or provider of the application for multipurpose data receiving device 130 can provide information and programming necessary for the devices to securely communicate through secured programming and updates. For example, the manufacturer can provide information that can be used to generate encryption keys for each device, including secured root keys for the analyte sensor 110 and optionally for the data receiving device 120 that can be used in combination with device-specific information and operational data (e.g., entropy-based random values) to generate encryption values unique to the device, session, or data transmission as need.

Analyte data associated with a user is sensitive data at least in part because this information can be used for a variety of purposes, including for health monitoring and medication dosing decisions. In addition to user data, the analyte monitoring system 100 can enforce security hardening against efforts by outside parties to reverse-engineering.

Communication connections can be encrypted using a device-unique or session-unique encryption key. Encrypted communications or unencrypted communications between any two devices can be verified with transmission integrity checks built into the communications. Analyte sensor 110 operations can be protected from tampering by restricting access to read and write functions to the memory 5020 via a communication interface. The sensor can be configured to grant access only to known or "trusted" devices, provided in a "whitelist" or only to devices that can provide a predetermined code associated with the manufacturer or an otherwise authenticated user. A whitelist can represent an exclusive range, meaning that no connection identifiers besides those included in the whitelist will be used, or a preferred range, in which the whitelist is searched first, but other devices can still be used. The sensor 110 can further deny and shut down connection requests if the requestor cannot complete a login procedure over a communication interface within a predetermined period of time (e.g., within four seconds). These characteristics safeguard against specific denial of service attacks, and in particular against denial of service attacks on a BLE interface.

As embodied herein, the analyte monitoring system 100 can employ periodic key rotation to further reduce the likelihood of key compromise and exploitation. A key rotation strategy employed by the analyte monitoring system 100 can be designed to support backward compatibility of field-deployed or distributed devices. As an example, the analyte monitoring system 100 can employ keys for downstream devices (e.g., devices that are in the field or cannot be feasibly provided updates) that are designed to be compatible with multiple generations of keys used by upstream devices.

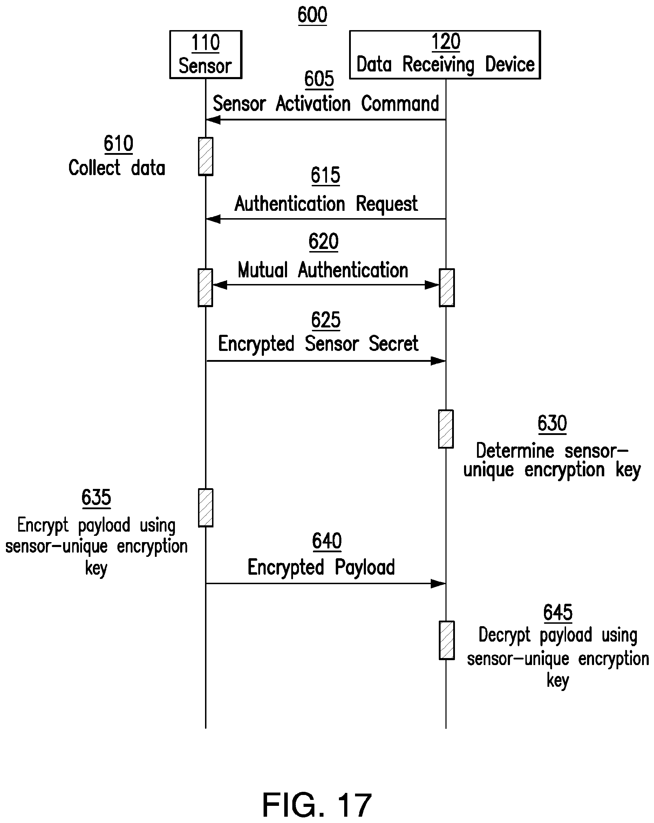

For purpose of illustration and not limitation, reference is made to the exemplary embodiment of a message sequence diagram 600 for use with the disclosed subject matter as shown in FIG. 17 and demonstrating an example exchange of data between a pair of devices, particularly a sensor 110 and a data receiving device 120. The data receiving device 120 can, as embodied herein, be a data receiving device 120 or a multi-purpose data receiving device 130. At step 605, the data receiving device 120 can transmit a sensor activation command 605 to the sensor 110, for example via a short-range communication protocol. The sensor 110 can, prior to step 605 be in a primarily dormant state, preserving its battery until full activation is needed. After activation during step 610, the sensor 110 can collect data or perform other operations as appropriate to the sensing hardware 5060 of the sensor 110. At step 615 the data receiving device 120 can initiate an authentication request command 615. In response to the authentication request command 615, both the sensor 110 and data receiving device 120 can engage in a mutual authentication process 620. The mutual authentication process 620 can involve the transfer of data, including challenge parameters that allow the sensor 110 and data receiving device 120 to ensure that the other device is sufficiently capable of adhering to an agreed-upon security framework described herein. Mutual authentication can be based on mechanisms for authentication of two or more entities to each other with or without on-line trusted third parties to verify establishment of a secret key via challenge-response. Mutual authentication can be performed using two-, three-, four-, or five-pass authentication, or similar versions thereof.

Following a successful mutual authentication process 620, at step 625 the sensor 110 can provide the data receiving device 120 with a sensor secret 625. The sensor secret can contain sensor-unique values and be derived from random values generated during manufacture. The sensor secret can be encrypted prior to or during transmission to prevent third-parties from accessing the secret. The sensor secret 625 can be encrypted via one or more of the keys generated by or in response to the mutual authentication process 620. At step 630, the data receiving device 120 can derive a sensor-unique encryption key from the sensor secret. The sensor-unique encryption key can further be session-unique. As such, the sensor-unique encryption key can be determined by each device without being transmitted between the sensor 110 or data receiving device 120. At step 635, the sensor 110 can encrypt data to be included in payload. At step 640, the sensor 110 can transmit the encrypted payload 640 to the data receiving device 120 using the communication link established between the appropriate communication models of the sensor 110 and data receiving device 120. At step 645, the data receiving device 120 can decrypt the payload using the sensor-unique encryption key derived during step 630. Following step 645, the sensor 110 can deliver additional (including newly collected) data and the data receiving device 120 can process the received data appropriately.

As discussed herein, the sensor 110 can be a device with restricted processing power, battery supply, and storage. The encryption techniques used by the sensor 110 (e.g., the cipher algorithm or the choice of implementation of the algorithm) can be selected based at least in part on these restrictions. The data receiving device 120 can be a more powerful device with fewer restrictions of this nature. Therefore, the data receiving device 120 can employ more sophisticated, computationally intense encryption techniques, such as cipher algorithms and implementations.

The analyte sensor 110 can be configured to alter its discoverability behavior to attempt to increase the probability of the receiving device receiving an appropriate data packet and/or provide an acknowledgement signal or otherwise reduce restrictions that can be causing an inability to receive an acknowledgement signal. Altering the discoverability behavior of the analyte sensor 110 can include, for example and without limitation, altering the frequency at which connection data is included in a data packet, altering how frequently data packets are transmitted generally, lengthening or shortening the broadcast window for data packets, altering the amount of time that the analyte sensor 110 listens for acknowledgement or scan signals after broadcasting, including directed transmissions to one or more devices (e.g., through one or more attempted transmissions) that have previously communicated with the analyte sensor 110 and/or to one or more devices on a whitelist, altering a transmission power associated with the communication module when broadcasting the data packets (e.g., to increase the range of the broadcast or decrease energy consumed and extend the life of the battery of the analyte sensor), altering the rate of preparing and broadcasting data packets, or a combination of one or more other alterations. Additionally, or alternatively, the receiving device can similarly adjust parameters relating to the listening behavior of the device to increase the likelihood of receiving a data packet including connection data.

As embodied herein, the analyte sensor 110 can be configured to broadcast data packets using two types of windows. The first window refers to the rate at which the analyte sensor 110 is configured to operate the communication hardware. The second window refers to the rate at which the analyte sensor 110 is configured to be actively transmitting data packets (e.g., broadcasting). As an example, the first window can indicate that the analyte sensor 110 operates the communication hardware to send and/or receive data packets (including connection data) during the first 2 seconds of each 60 second period. The second window can indicate that, during each 2 second window, the analyte sensor 110 transmits a data packet every 60 milliseconds. The rest of the time during the 2 second window, the analyte sensor 110 is scanning. The analyte sensor 110 can lengthen or shorten either window to modify the discoverability behavior of the analyte sensor 110.

In particular embodiments, the discoverability behavior of the analyte sensor can be stored in a discoverability profile, and alterations can be made based on one or more factors, such as the status of the analyte sensor 110 and/or by applying rules based on the status of the analyte sensor 110. For example, when the battery level of the analyte sensor 110 is below a certain amount, the rules can cause the analyte sensor 110 to decrease the power consumed by the broadcast process. As another example, configuration settings associated with broadcasting or otherwise transmitting packets can be adjusted based on the ambient temperature, the temperature of the analyte sensor 110, or the temperature of certain components of communication hardware of the analyte sensor 110. In addition to modifying the transmission power, other parameters associated with the transmission capabilities or processes of the communication hardware of the analyte sensor 110 can be modified, including, but not limited to, transmission rate, frequency, and timing. As another example, when the analyte data indicates that the subject is, or is about to be, experiencing a negative health event, the rules can cause the analyte sensor 110 to increase its discoverability to alert the receiving device of the negative health event.

As embodied herein, certain calibration features for the sensing hardware 5060 of the analyte sensor 110 can be adjusted based on external or interval environment features as well as to compensate for the decay of the sensing hardware 5060 during expended period of disuse (e.g., a "shelf time" prior to use). The calibration features of the sensing hardware 5060 can be autonomously adjusted by the sensor 110 (e.g., by operation of the ASIC 5000 to modify features in the memory 5020 or storage 5030) or can be adjusted by other devices of the analyte monitoring system 100.

As an example, sensor sensitivity of the sensing hardware 5060 can be adjusted based on external temperature data or the time since manufacture. When external temperatures are monitored during the storage of the sensors, the disclosed subject matter can adaptively change the compensation to sensor sensitivity over time when the device experiences changing storage conditions. For purpose of illustration not limitations, adaptive sensitivity adjustment can be performed in an "active" storage mode where the analyte sensor 110 wakes up periodically to measure temperature. These features can save the battery of the analyte device and extend the lifespan of the analyte sensors. At each temperature measurement, the analyte sensor 110 can calculate a sensitivity adjustment for that time period based on the measured temperature. Then, the temperature-weighted adjustments can be accumulated over the active storage mode period to calculate a total sensor sensitivity adjustment value at the end of the active storage mode (e.g., at insertion). Similarly, at insertion, the sensor 110 can determine the time difference between manufacture of the sensor 110 (which can be written to the storage 5030 of the ASIC 5000) or the sensing hardware 5060 and modify sensor sensitivity or other calibration features according to one or more known decay rates or formulas.

Additionally, for purpose of illustration and not limitation, as embodied herein, sensor sensitivity adjustments can account for other sensor conditions, such as sensor drift. Sensor sensitivity adjustments can be hardcoded into the sensor 110 during manufacture, for example in the case of sensor drift, based on an estimate of how much an average sensor would drift. Sensor 110 can use a calibration function that has time-varying functions for sensor offset and gain, which can account for drift over a wear period of the sensor. Thus, sensor 110 can utilize a function used to transform an interstitial current to interstitial glucose utilizing device-dependent functions describing sensor 110 drift over time, and which can represent sensor sensitivity, and can be device specific, combined with a baseline of the glucose profile. Such functions to account for sensor sensitivity and drift can improve sensor 110 accuracy over a wear period and without involving user calibration.

The sensor 110 detects raw measurement values from sensing hardware 5060. On-sensor processing can be performed, such as by one or more models trained to interpret the raw measurement values. Models can be machine learned models trained off-device to detect, predict, or interpret the raw measurement values to detect, predict, or interpret the levels of one or more analytes. Additional trained models can operate on the output of the machine learning models trained to interact with raw measurement values. As an example, models can be used to detect, predict, or recommend events based on the raw measurements and type of analyte(s) detected by the sensing hardware 5060. Events can include, initiation or completion of physical activity, meals, application of medical treatment or medication, emergent health events, and other events of a similar nature.

Models can be provided to the sensor 110, data receiving device 120, or multi-purpose data receiving device 130 during manufacture or during firmware or software updates. Models can be periodically refined, such as by the manufacturer of the sensor 110 or the operator of the analyte monitoring system 100, based on data received from the sensor 110 and data receiving devices of an individual user or multiple users collectively. In certain embodiments, the sensor 110 includes sufficient computational components to assist with further training or refinement of the machine learned models, such as based on unique features of the user to which the sensor 110 is attached. Machine learning models can include, by way of example and not limitation, models trained using or encompassing decision tree analysis, gradient boosting, ada boosting, artificial neural networks or variants thereof, linear discriminant analysis, nearest neighbor analysis, support vector machines, supervised or unsupervised classification, and others. The models can also include algorithmic or rules-based models in addition to machine learned models. Model-based processing can be performed by other devices, including the data receiving device 120 or multi-purpose data receiving device 130, upon receiving data from the sensor 110 (or other downstream devices).

Data transmitted between the sensor 110 and a data receiving device 120 can include raw or processed measurement values. Data transmitted between the sensor 110 and data receiving device 120 can further include alarms or notification for display to a user. The data receiving device 120 can display or otherwise convey notifications to the user based on the raw or processed measurement values or can display alarms when received from the sensor 110. Alarms that may be triggered for display to the user include alarms based on direct analyte values (e.g., one-time reading exceeding a threshold or failing to satisfy a threshold), analyte value trends (e.g., average reading over a set period of time exceeding a threshold or failing to satisfy a threshold; slope); analyte value predictions (e.g., algorithmic calculation based on analyte values exceeds a threshold or fails to satisfy a threshold), sensor alerts (e.g., suspected malfunction detected), communication alerts (e.g., no communication between sensor 110 and data receiving device 120 for a threshold period of time; unknown device attempting or failing to initiate a communication session with the sensor 110), reminders (e.g., reminder to charge data receiving device 120; reminder to take a medication or perform other activity), and other alerts of a similar nature. For purpose of illustration and not limitation, as embodied herein, the alarm parameters described herein can be configurable by a user or can be fixed during manufacture, or combinations of user-settable and non-user-settable parameters.

According to aspects of the disclosed subject matter, an in vivo analyte sensor and method for detecting alcohol levels, including but limited to ethanol levels, is provided.

As used herein, the term "alcohol," and grammatical variants thereof, refers to any primary, secondary, or tertiary alcohol. For example, the alcohol sensors of the present disclosure can detect ethanol, methanol, butanol, propanol, isopropyl alcohol, and the like, and any combination thereof.

The term "reference electrode" as used herein, can refer to either reference electrodes or electrodes that function as both, a reference and a counter electrode. Similarly, the term "counter electrode," as used herein, can refer to both, a counter electrode and a counter electrode that also functions as a reference electrode.

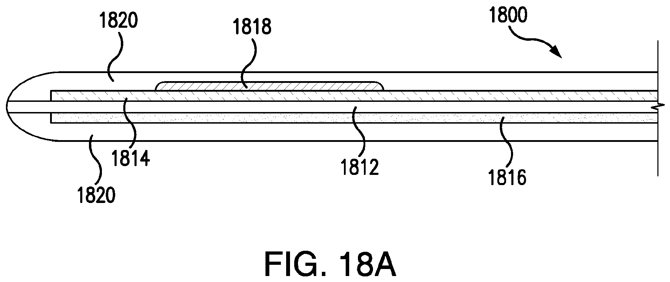

The sensor 110 described herein can include a sensing element that includes one or more electrodes configured to detect one or more analyte levels in a bodily fluid, examples of which are shown in FIGS. 18A-20. The one or more electrodes can include one or more enzyme response elements. For example, and as embodied herein, sensor 110 can detect alcohol levels in a bodily fluid. In another example, sensor 110 can detect alcohol and glucose levels in a bodily fluid. In yet another example, sensor 110 can detect alcohol and ketone levels in a bodily fluid. Sensor 110 can include one working electrode capable of detecting alcohol and/or another working electrode capable of detecting glucose, ketone, lactate, and/or any other analyte levels. In some examples, sensor 110 can include two or more sensing elements configured to detect two or more analyte levels in a bodily fluid. Sensor 110 can also be referred to as an "analyte sensor."

*In vivo* alcohol concentration of an individual can change dramatically based on alcohol consumption or a variety of physiological factors. For example, alcohol can be metabolized by individuals at different rates, which can cause alcohol levels to vary between individuals consuming the same dose of alcohol per body weight. The equilibrium concentration of alcohol depends on at least water content, rate of blood flow, and body mass. Given that alcohol passes through biological membranes, alcohol can flow from the bloodstream to all tissues and fluids. Said flow can be proportional to the water content of the tissues and fluids. Further, as described above, alcohol concentration can influence the function of one or more other analytes of an individual, thereby influencing the health or physiological condition of an individual. For example, any of the sensor systems and analyte sensor configurations described hereinafter can feature one or more enzymes used to detect alcohol.

*Ex vivo* alcohol measurements can be performed by taking physical blood samples, urine samples, saliva samples, perspiration or sweat samples, or breath tests. These measurements, however, are static in time and, in some instances, can reflect false or inaccurate results. Analyte sensors, on the other hand, are dynamic and updated over time. Alcohol sensors are responsive to *in vivo* alcohol levels and are capable of providing "continuous" measurement. Alcohol sensors can provide a plurality of alcohol concentration measurements over a continuous period of time, such as seconds, minutes, or hours to days, weeks, or months.

An individual wearing a continuous alcohol sensor can access real-time alcohol level information to make various decisions based thereon, such as whether to operate a vehicle, and/or whether other analyte levels, such as glucose, are dysregulated based on the alcohol level. For example, the alcohol sensor can be used to monitor, test, and/or evaluate alcohol levels in individuals suffering from alcohol misuse, abuse, or addiction. The alcohol levels can be monitored by individuals themselves, by health practitioners, or by law enforcement professionals.

A display unit of a sensor or a reader device can be used to provide direction, suggestion, guidance, recommendations, and/or any other output associated with or corresponding to the alcohol concentrations. Suitable processing algorithms, processors, memory, electronic components, and the like can reside in any of a trusted computer system, remote terminal, cloud server, reader device, and/or a housing for the sensor itself. Guidance, recommendations, output, and/or the like can be shown on a display unit or graphical user interface that is in electronic communication with the sensor or one or more components of the sensor system. The display unit or device can be a dedicated reader device or a user equipment, such as mobile device. Alternately, the display unit or device can be a third-party server, cloud server, or remote terminal that communicates with various software applications, which can be accessed by medical professionals. The dedicated reader device, a user equipment, or one of the servers can further relay the data or output to one or more secondary devices such as smart home devices, wearable watches or devices, personal health monitors, or the like.

As embodied herein, the reader device can include a processor, memory, an input/output interface, and a communication interface. Processor includes hardware for executing instructions, such as those making up a computer program. As an example and not by way of limitation, to execute instructions, processor can retrieve (or fetch) the instructions from an internal register, an internal cache, memory, or storage; decode and execute them; and then write one or more results to an internal register, an internal cache, memory, or storage. Processor can also include one or more internal caches for data, instructions, or addresses. The one or more processors can include one or more arithmetic logic units (ALUs) or be a multi-core processor.

As embodied herein, memory includes main memory for storing instructions for processor to execute or data for processor to operate on. As an example, and not by way of limitation, reader device can load instructions from storage or another source to memory. Processor can then load the instructions from memory to an internal register or internal cache. To execute the instructions, processor can retrieve the instructions from the internal register or internal cache and decode them. During or after execution of the instructions, processor can write one or more results (which can be intermediate or final results) to the internal register or internal cache. Processor can then write one or more of those results to memory. For example, the memory can include a random access memory (RAM). This RAM can be volatile memory, a dynamic RAM (DRAM), or static RAM (SRAM). The RAM can be single-ported or multi-ported RAM, and the memory can include one or more memories.

As described herein, with reference to FIG. 1A, sensor 110 can be at least partially inserted into the dermal or subcutaneous layer of the skin. Sensor 110 can include a sensor tail of sufficient length for insertion to a desired depth in the interstitial fluid. The sensor tail can include at least one working electrode and one or more active areas (sensing regions/spots or sensing layers) located thereon that are active for sensing alcohol (or in some instances one or more additional analytes). For example, the active areas can be in the form of one or more discrete spots. The number of discrete spots, for example, can range from one to a dozen spots. The one or more discrete spots can range from about 0.01 millimeters squared (mm2) to about 1.00 mm2, such as from about 0.1 mm2 to about 0.5 mm2, about 0.25 mm2 to about 0.75 mm2, about 0.05 mm2 to about 0.2 mm2, or have any other smaller or larger value.

The one or more active areas can include one or more enzymes used to facilitate the detection of alcohol. The active areas, for example, can include a polymeric material to which one or more of the enzymes are chemically bonded (e.g., covalently bonded, ionically bonded, and the like) or otherwise immobilized (e.g., unbound in a matrix). For example, each active area can be overcoated with a mass-limiting, bio-compatibility membrane, and/or an electron transfer agent to facilitate detection of at least alcohol.

As embodied herein, alcohol levels can be monitored in any biological fluid of interest such as dermal fluid, interstitial fluid, plasma, blood, lymph, synovial fluid, cerebrospinal fluid, saliva, bronchoalveolar lavage, amniotic fluid, or the like.

As illustrated in FIG. 1A, sensor control unit 102 can manually or automatically forward data obtained with sensor 110 to reader device 120. For example, alcohol concentration data can be communicated automatically or periodically after a certain time period has passed, with the data being stored in a memory until transmittal (e.g., every several seconds, every minute, five minutes, or other predetermined time period). Sensor control unit 102 can also communicate with reader device 120 according to a non-set schedule based on a wearer or user action or request. For example, data can be communicated from sensor control unit 102 using NFC or RFID technology when the sensor electronics are brought into communication range of reader device 120. In addition to, or as an alternative, Bluetooth can be used to facilitate the communication of data from sensor control unit 102 to reader device 120. Until the data is communicated to reader device 120, the data can remain stored in a memory of sensor control device 102. In one example, the data can be stored for up to eight hours in the memory of sensor control device 102. In other examples, the data can be stored for up to 0.5 hours, 1 hour, 2 hours, 3 hours, 4 hours, 6 hours, 10 hours, 12 hours, 24 hours, or any other number of hours. The data can then be transmitted from sensor control device 102 to reader device 120 when sensor control device 102 is within a given distance from reader device 120.

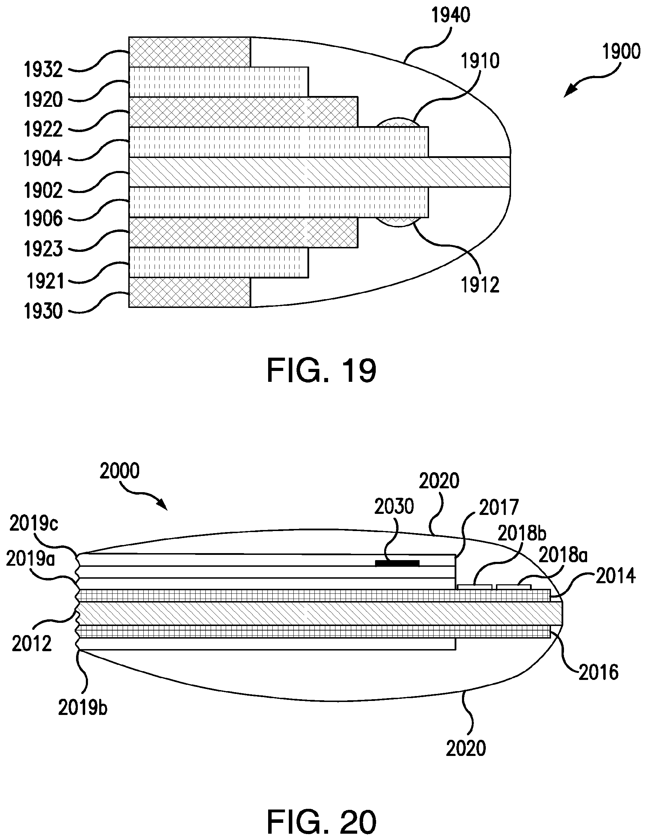

For purpose of illustration and not limitation, as embodied herein, exemplary alcohol sensors can include active areas located on one or both sides of a single working electrode or on one or both sides of two or more separate working electrodes. In other examples, alcohol sensors can employ one or more working electrodes and one or more other electrodes, such as a reference electrode. Sensor configurations having a single working electrode are described hereinafter with reference to FIGS. 18A-18C. Each of these sensor configurations can suitably incorporate one or more alcohol-responsive active areas. Sensor configurations having multiple working electrodes are described thereafter in reference to FIGS. 19 and 20. When multiple working electrodes are present, one or more alcohol-responsive active areas located on one or more of the multiple working electrodes, while one or more working electrodes can be used to detect another analyte of interest in concert with detection of alcohol levels.

When a single working electrode is included in an alcohol sensor, a counter electrode and a reference electrode can also be included in the alcohol sensor. The alcohol sensor, therefore, can include three total electrodes, with a single working electrode. For example, a working electrode and a second electrode, such as a counter electrode or a reference electrode, can be included. In one example the counter electrode and the reference electrode can be combined into one second electrode. In examples including two or three-electrodes, one or more active areas of the alcohol sensor can be in contact with the working electrode. The one or more active areas, for example, can include one or more enzymes.

The various electrodes can be at least partially stacked or layered on one another. For example, the various electrodes can be laterally spaced apart from one another on the sensor tail. Similarly, the associated active areas upon each electrode can be stacked vertically on top of one another or can be laterally spaced apart. The various electrodes can be electrically isolated from one another by a dielectric material or similar insulator.

FIG. 18A is a cross-sectional diagram illustrating an exemplary analyte sensor including a single active area as embodied herein. For example, FIG. 18A illustrates a two-electrode sensor configuration. The analyte sensor shown in FIG. 18A, for example, can detect at least alcohol levels. Sensor 1800 of FIG. 18A can be similar to sensor 110 shown in FIG. 1A. Sensor 1800 can include substrate 1812 disposed between working electrode 1814 and counter/reference electrode 1816. In some examples, working electrode 1814 and counter/reference electrode 1816 can be located on the same side of substrate 1812 (e.g., top or bottom) with a dielectric material interposed in between. Active area 1818 can be disposed as one or more layers on a portion of working electrode 1814. Further, active area 1818 can include a single spot or multiple spots configured for detection of one or more analytes of interest. One or more enzymes can be on the single spot or the multiple spots of active area 1818.

In addition, as shown in FIG. 18A membrane 1820 can overcoat at least active area 1818. Membrane 1820 can also overcoat some or all of working electrode 1814, counter/reference electrode 1816, or entire analyte sensor 1800. One or both faces of analyte sensor 1800 can be overcoated with membrane 1820. Membrane 1820 can include one or more polymeric membrane materials that can limit the analyte flux to active area 1818. For example, sensor 1800 can assay alcohol by using at least one of coulometric, amperometric, voltammetric, potentiometric electrochemical, or iontophoretic (including reverse iontophoresis) detection.

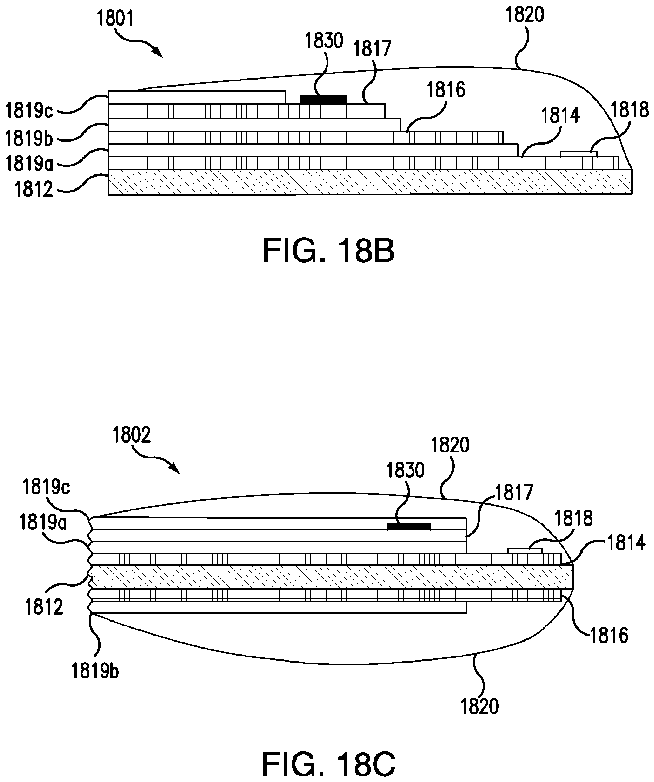

FIG. 18B is a cross-sectional diagram illustrating an exemplary analyte sensor including a single active area as embodied herein. FIG. 18C is a cross-sectional diagram illustrating an exemplary analyte sensor including a single active area as embodied herein. For example, FIGS. 18B and 18C illustrate a three-electrode sensor configuration. Both FIGS. 18B and 18C show working electrode 1814, a counter electrode or reference electrode 1816, and an additional electrode 1817. Additional electrode 1817 can be either another counter/reference electrode or another working electrode. Additional electrode 1817 can be disposed upon either working electrode 1814 or counter/reference electrode 1816, with a separating layer of dielectric material placed therebetween. As depicted in FIG. 18B, dielectric layers 1819a, 1819b, and 1819c separate electrodes 1814, 1816, and 1817 from one another to provide electrical isolation. As depicted in FIG. 18C, on the other hand, at least one of electrodes 1814, 1816, and 1817 can be located on opposite faces of substrate 1812. Therefore, working electrode 1814 and counter electrode 1816 can be located on opposite faces of substrate 1812, with reference electrode 1817 being located on one of electrodes 1814 or 1816 and spaced apart therefrom with dielectric materials.

As embodied herein, working electrode 1814 and reference electrode 1816 can be located upon opposite faces of substrate 1812, with counter electrode 1817 being located upon one of electrodes 1814 or 1816 and spaced apart therefrom with a dielectric material. In yet another embodiment, the reference or counter electrode 1816 can be located on one face of the substrate 1812 and working electrode 1814 on the opposite face. A reference material layer 1830, which can be composed of silver (Ag) or silver chloride (AgCl) can be present on electrode 1817. Reference material layer 1830 can be located in any other location on electrode 1817, electrode 1814, or electrode 1816.

As shown in FIGS. 18B and 18C, analyte sensors 1801 and 1802 can comprise one or more enzymes in active area 1818. In some examples, active area 1818 can include multiple spots or a single spot configured for detection of at least alcohol. Analyte sensors 1801 and 1802, for example, can assay alcohol, or one or more additional analytes, via coulometric, amperometric, voltammetric, potentiometric electrochemical, or iontophoretic detection techniques.

With continued reference to FIGS. 18B and 18C, membrane 1820 can overcoat active area 1818, as well as other sensor components, in sensors 1801 and 1802. Additional electrode 1817 can also be overcoated with membrane 1820. While FIGS. 18B and 18C have depicted all of electrodes 1814, 1816, and 1817 as being overcoated with membrane 1820, in other examples only working electrode 1814 can be overcoated or only working electrode 1814 and one other electrode can be overcoated. The thickness of membrane 1820 at each of electrodes 1814, 1816, and/or 1817 can be the same or different. For example, the amount of surface area of each of electrodes 1814, 1816, and/or 1817 that membrane 1820 overcoats can be the same or different. One or both faces of analyte sensors 1801 and 1802 can be overcoated with membrane 1820. Alternatively, the entirety of analyte sensors 1801 and 1802 can be overcoated.

FIG. 19 is a cross-sectional diagram illustrating an exemplary analyte sensor including two active areas as embodied herein. As shown in FIG. 19, alcohol sensor 1900 has two working electrodes, a reference electrode, and a counter electrode. Sensor 1900 includes working electrodes 1904 and 1906 disposed upon opposite faces of substrate 1902. Active area 1910 is disposed on the surface of working electrode 1904, while active area 1912 is disposed on the surface of working electrode 1906. One or more enzymes configured to detect alcohol can be present in active areas 1910 and 1912. For example, one or more active areas 1910 or 1912 can be configured to detect alcohol concentration and another analyte of interest, such as glucose, lactate, or ketone. Counter electrode 1920 can be electrically isolated from working electrode 1904 by dielectric layer 1922, while reference electrode 1921 can be electrically isolated from working electrode 1906 by dielectric layer 1923. Outer dielectric layers 1930 and 1932 are positioned on reference electrode 1921 and counter electrode 1920, respectively. Membrane 1940 can overcoat at least active areas 1910 and 1912. Other components of analyte sensor 1900 can be overcoated with membrane 1940, and/or one or both faces of analyte sensor 1900, or a portion thereof, can be overcoated with membrane 1940. Similar to analyte sensors 1800, 1801, and 1802 shown in FIGS. 18A-18C, sensor 1900 can be operable for assaying at least alcohol via an coulometric, amperometric, voltammetric, potentiometric electrochemical, or iontophoretic technique.

As embodied herein, alternative sensor configurations, differing from those shown in FIG. 19, can include multiple working electrodes and a combined counter/reference electrode, instead of separate counter and reference electrodes 1920 and 1921. In other examples, the positioning of counter electrode 1920 and reference electrode 1921 can be reversed from that depicted in FIG. 19. Further, working electrodes 1904 and 1906 can reside on the same side of substrate 1902.

Although the above description pertaining to FIGS. 18A-C and 19 is primarily directed to analyte sensor configurations having one or two working electrodes, in other examples more than two working electrodes can be used by the analyte sensor. Additional working electrodes can provide for additional active areas and corresponding sensing capabilities.

Further, while FIGS. 18A-18C and 19 show planar substrates (e.g., substantially flat) having electrodes and active areas disposed thereon, the analyte sensor can have various other configurations. For example, the substrate can be substantially non-planar (e.g., curved, semi-hemispherical, or spherical), cylindrical, helical, otherwise irregular in shape, or any combination thereof. Similarly, the one or more electrodes can be substantially non-planar (e.g., relatively curved, semi-hemispherical, or spherical), cylindrical, helical, otherwise irregular in shape, or any combination thereof. The electrodes can be arranged in layers, concentrically, or in any other arrangement. Sensing regions disposed on a working electrode can cover at least a portion of a working electrode as a single layer or as discrete regions of various shapes, such as square, circular, semicircular, arcuate, rectangular, polygonal, or otherwise irregular.

As embodied herein, an electron transfer agent can be present in one or more of the active areas of the alcohol sensors. Suitable electron transfer agents can help to facilitate conveyance of electrons to the working electrode when an alcohol analyte undergoes an oxidation-reduction reaction. The electron transfer agent within each active area can dictate the oxidation-reduction potential observed for the alcohol analyte.

FIG. 20 is a cross-sectional diagram illustrating an exemplary analyte sensor including two active areas as embodied herein. The analyte sensor configuration of FIG. 20 can be similar to FIG. 18C, with FIG. 20 including two active areas 2018a, 2018b. As shown in FIG. 20, analyte sensor 2000 includes active areas 2018a and 2018b on the surface of working electrode 2014. Active area 2018a includes a first electron transfer agent and a first analyte-responsive enzyme bonded to active area 2018a. Active area 2018b similarly includes a second electron transfer agent and a second analyte-responsive enzyme bonded to active area 2018b. As embodied herein, the first and second electron transfer agents can differ in composition so as to provide separation of the oxidation-reduction potentials of first active area 2018a and second active area 2018b. For example, active area 2018b can comprise an alcohol-responsive enzyme, such as ketone reductase, while active area 2018a can comprise a glucose-responsive enzyme, such as glucose oxidase.

The oxidation-reduction potentials of first active area 2018a and second active area 2018b can be sufficiently separated to allow for a first independent signal to be produced by first active area 2018a, and a second independent signal to be produced by second active area 2018b. Accordingly, analyte sensor 2000 can operate at a first potential, at which an oxidation-reduction reaction occurs within first active area 2018a, but not within a second active area 2018b. A first analyte, such as glucose, can be selectively detected at or above the oxidation-reduction potential of first active area 2018a, provided that the applied potential is not high enough to promote an ketone reductase reaction with second active area 2018b. A concentration of the first analyte can be determined from the produced signal by referring to a lookup table or calibration curve.

Similarly, the oxidation-reduction potential of second active area 2018b can take place simultaneously or near simultaneously within both first active area 2018a and second active area 2018b. As a result, the signal produced at or above the oxidation-reduction potential of second active area 2018b can comprise a composite signal having signal contributions from both first active area 2018a and second active area 2018b. To determine the concentration of the second analyte, such as alcohol, from the composite signal, the signal from first active area 2018a at or above its corresponding oxidation-reduction potential can be subtracted from the composite signal to provide a difference signal associated with second active area 2018b alone. Once the difference signal associated with second active area 2018b is determined, the concentration of a second analyte can be determined using a lookup table or calibration curve.

The active area of the alcohol sensor, for example, can be based on an X7-wired diaphorase coupled to a ketone reductase (KRED) through freely diffusing nicotinamide-adenine dinucleotide phosphate (NADP) trapped within the sensing layer. Such an active area can exemplify low enzyme activity, which can improve sensor performance. Low enzyme activity, for example, can be about 10 to about 10,000 times less active than normal acting analyte sensors. For active areas with low enzyme activities, the innate thermostability of the enzyme can have an increasingly important effect on the rendered signal. Under low enzyme activity conditions, for example, it can be challenging to compensate enzyme instability by increasing the enzyme loading. This results in difficulty achieving a stable sensitivity (ratio of current to alcohol concentration) over the implant time period of the sensor and/or during the shelf storage life of the sensor. Stable sensitivity, for example, can be achieved by increasing the thermal stability of the enzyme and/or by use of a membrane-limited sensor. Use of the proposed peak width measurement can allow for alcohol quantification using data from a sensor, despite the difficulty of achieving a stable sensitivity. The implant time period can be several hours, days, weeks , or months. For example, the implant period can range from 2 hours to 14 days.

FIGS. 21A and 21B are graphs illustrating a current output of an exemplary analyte sensor as embodied herein. While FIGS. 21A and 21B illustrate the same current output, FIG. 21A illustrates the current output from day 1 (9/21) to day 14 (10/5), and FIG. 21B focuses on the current output from day 7 (9/27) to day 12 (10/2). The analyte sensor, for example, can be an ethanol sensor 2102. FIG. 21A illustrates a current output 2104 of ethanol sensor 2102 over a two-week period. Over the first 5 days of wearing ethanol sensor 2102, the individual wearing the sensor consumed varying quantities and strengths of alcoholic beverages, each time producing a discernible peak in the signal output produced by the alcohol sensor. This peak can be defined by a peak height or peak amplitude, used interchangeably herein. The peak signal output, for example, can be a current, voltage, charge, energy, electric-potential, potential-difference, or any other signal output. For example, on day 1 (9/21), the individual consumed 24 ounces of beer having a 7% alcohol content. The corresponding current output was about 5200 picoamps. On day 2 (9/22), the individual consumed 24 ounces of beer having a 5% alcohol content, while on day 3 (9/23), the individual consumed 24 ounces of beer having a 7% alcohol content. The corresponding current output was about 5100 picoamps and around 3700 picoamps, respectively. On day 4 (9/24), the individual wearing the sensor does not consume any alcohol, while on day 5 (9/25), the individual consumed 16 ounces of beer having a 7% alcohol content. The corresponding current output for day 5 was about 3200 picoamps.

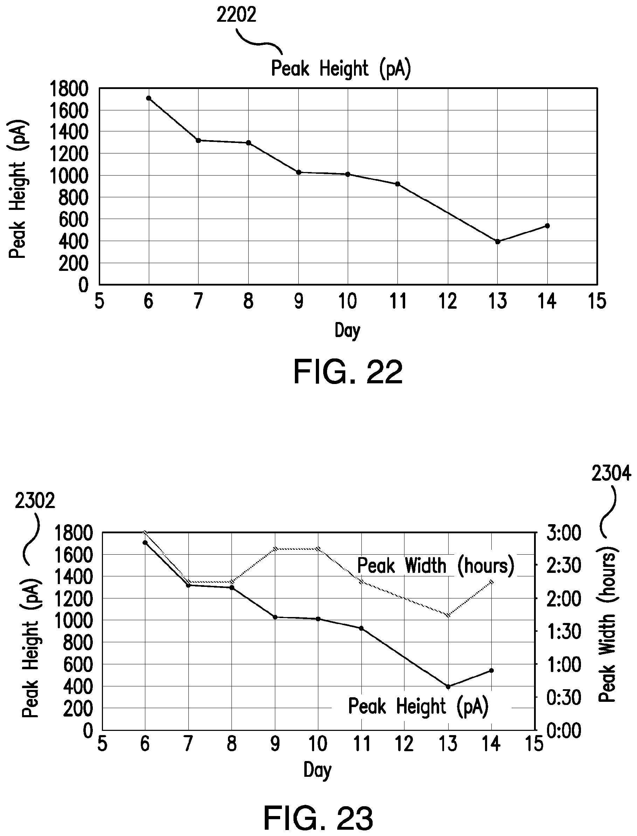

For the remaining life period of the sensor, days 6-14 (9/26-10/5), the individual consumed 12 ounces of beer having a 7% alcohol content each day, except for day 12 when no alcohol was consumed. While the amount and content of alcohol remained consistent between days 6-14, the current output varied from about 2800 picoamps on day 6 to about 1100 picoamps on day 14. As shown, the current output steadily decreased or decayed from day 6 to day 14. This decay can be seen not only in FIG. 21A, but also in current output depicted (after subtraction of background current not due to alcohol consumption) in FIG. 22.

FIG. 22 is a graph illustrating a current output of an exemplary analyte sensor as embodied herein. As illustrated in FIG. 22, peak current heights 2202 of the alcohol sensor are the same as the peak current heights for days 6 to 14 of FIG. 21A, with the threshold or background signal levels having been subtracted or removed. The threshold or background signal levels can also be referred to as noise levels. An example threshold or background signal level 2106 is illustrated in FIG. 21A. As shown in FIG. 21A, threshold or background signal level hovers around 1000 picoamps for days 1-3, and then linearly declines to about 500 picoamps on day 14. The threshold or background signal level, for example, can either linearly or non-linearly decline during at least a portion of the lifetime of the analyte sensor. The threshold or background signal level, for example, can also either linearly or non-linearly incline during at least a portion of the lifetime of the analyte sensor. The threshold or background signal level can stabilize or balance after an initial wear period. The initial wear period, for example, can be 2 days, 3 days, 4 days, 5 days, 6 days, or 7 days. The threshold or background signal level, for example, can correspond to the charge passed in an electrochemical assay in the absence of an analyte.

As shown in FIG. 22, the peak height and area decays over the 9 day period, during which the individual consumed a consistent amount of alcohol. On day 6, for example, the peak height is about 1700 picoamps, while in day 14 the peak height is about 550 picoamps. The peak height of the current output, however, reaches a low of 400 picoamps on day 13. As such, the overall decrease of the peak height, from the highest to the lowest value, can be about 75%, and which can occur, as embodied herein, when the individual consumes a consistent amount of alcohol each day. The decay, at least in part, can be caused by the enzyme degradation of the active areas on the alcohol sensor. While in FIG. 21A the peak height on day 13 appears higher than day 14, in FIG. 22 the peak height on day 13 appears lower than day 14. The lower peak height on day 13 in FIG. 22 can be the results of a higher threshold or background signal level on day 13 than on day 14.

Determining blood alcohol concentration (BAC, also referred to as blood alcohol content) based on factors other than, or in addition to, peak height can improve accuracy of such determination, due at least in part to the decay in peak height over time. For example and without limitation, as embodied herein, an analyte sensor can determine BAC by accounting for peak signal width in a current signal over time. The peak signal width can be the amount of time that the signal exceeds a threshold signal level, a background signal level, or a noise level. The amount of time can be measured in minutes, hours, or days. The calculation of the peak signal can be performed either at sensing system 100 or reader device 120. For example, as shown in FIG. 21B, the peak signal width on day 10 (9/30) crosses threshold or background signal level 2106 at time 2108 and time 2110. The peak signal width, therefore, spans from time 2108 to time 2110, which can be about 2 hours and fifteen minutes.

Peak signal width of an alcohol analyte measurement can be correlated to other aspects of alcohol metabolism. For example, the rate of alcohol elimination, meaning the rate in which alcohol is metabolized or absorbed by the human body, can be constant for a given individual, meaning that the rate can be independent of the amount of alcohol consumed. While other drugs can have an elimination rate proportional to drug concentration, and thus having a half-life elimination pattern, alcohol metabolism or elimination rate can be considered independent of the amount of alcohol consumed. As such, BAC declines at a linear rate, such that the peak width can be proportional to peak.

FIG. 23 is a graph illustrating a current output of an exemplary analyte sensor as embodied herein. FIG. 23, for example, illustrates a comparison between the peak width in hours 2304 versus the peak height in picoamps 2302 of the current output of the alcohol sensor. As shown in FIG. 23, the peak width 2304 starts at about 3 hours and decreases to about 2 hours, which is roughly a 33% decay. On the other hand, the peak height 2302 starts at a peak of about 1800 picoamps and decreases to about 400 picoamps, which is roughly a 75% decay. Accordingly, the BAC can be determined by accounting for peak signal width. Additionally, for purpose of illustration only and not limitation, BAC can be determined using peak amplitude, in combination with peak width.

As discussed herein, the alcohol elimination rate can vary amongst individuals. The relationship between peak width and BAC can also vary amongst individuals. For example, BAC determinations can be adjusted for such variations by combining the peak width information with peak amplitude information, for example and without limitation during a calibration period, which can be an initial wear period. For example, the peak width and amplitude of an individual can be measured during the first 10 days, 6 days, 5 days, 4 days, 3 days, 2 days, or 1 day of the wear period. The peak width and amplitude of an individual can be measured within the first 24-hour wear period, for example, within the first 12 hours, 6 hours, 3 hours, 2 hours, or 1 hour. By capturing data during the initial wear period, the peak signal width and amplitude of an individual can be captured, while minimizing or eliminating the effect of enzyme degradation on the measured peak width and amplitude. These peak signal width and/or peak signal amplitude measured in the initial wear period can be referred to as an early peak signal width or early peak signal amplitude.

For example, the BAC can be determined using the following equation: $BAC = K1 \times i_{peak}$, where $BAC$ is the blood alcohol concentration, $K1$ is the factory sensitivity for the alcohol sensor determined from a calibration, and $i_{peak}$ is the peak signal. The calibration of the alcohol sensor can occur during manufacturing or design stages of the alcohol sensor. For example, the calibration can be a beaker calibration. The factory sensitivity, for example, can be a predetermined number or factor that is associated with a given manufacturing batch or alcohol sensor. An individual wearing the alcohol sensor can enter an identification or code associated with the alcohol sensor into the reader device, and the factory sensitivity corresponding to the alcohol sensor can be applied to the signal produced by the alcohol sensor. Alternatively, the factory sensitivity can be programmed into the alcohol sensor itself without further input from the wearer or another user. The blood alcohol concentration determination can thus be adjusted based on the sensitivity of each individual alcohol sensor or a manufacturing batch of alcohol sensors, for purpose of illustration and not limitation.

As embodied here, the peak signal can be determined based on the following equation: $i_{peak} = K2 \times Width_{peak}$, where $K2$ is the ratio of peak signal height or amplitude to peak signal width, $i_{peak}$ is the peak signal, and $Width_{peak}$ is the peak signal width. Once the $K1$ and $K2$ are determined during the initial wear period, the BAC for subsequent peaks, including peaks effected by enzyme degradation of the alcohol sensor, is determined using the following equation: $BAC = K1 \times K2 \times Width_{peak}$. A BAC determined based on the $K1$ and $K2$ can result in an improved, more stable BAC level, rather than solely relying on peak signal amplitude.

Therefore, the blood alcohol concentration can be determined, in part, based on the peak signal width. The blood alcohol concentration can also be determined based on a ratio of a peak signal amplitude to the peak signal width.

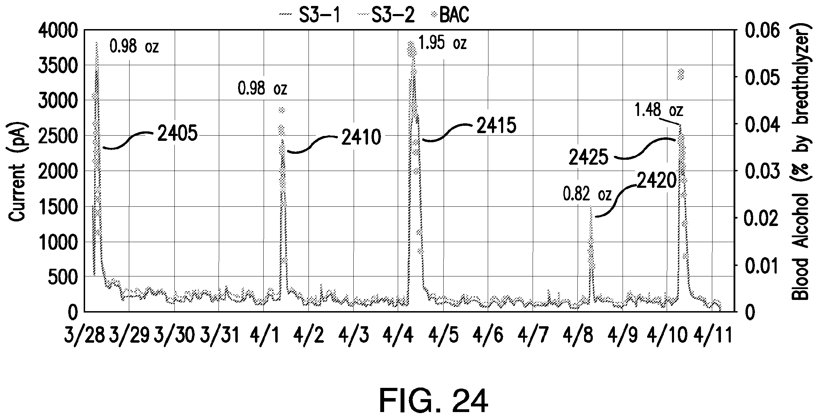

FIG. 24 is a graph illustrating current outputs of two exemplary analyte sensor as embodied herein worn simultaneously. FIG. 24 illustrates the current outputs of the two sensors from day 1 (3/28) to day 15 (4/11), as well as corresponding blood alcohol concentrations (BACs). The analyte sensors, for example, can be two ethanol sensors 2102. FIG. 24 illustrates a current output S3-1 of a first ethanol sensor and a current output S3-2 of a second ethanol sensor over the fifteen-day period. As illustrated by FIG. 24, the outputs S3-1 and S3-2 show consistent patterns in detecting alcohol.

During the wearing periods of the two ethanol sensors, the individual wearing the sensors consumed varying quantities of alcoholic beverages, each time producing a discernible peak in the signal output. This peak can be defined by a peak height or peak amplitude, used interchangeably herein. The peak signal output, for example, can be a current, voltage, charge, energy, electric-potential, potential-difference, or any other signal output. For purpose of illustration not limitation, on day 1 (3/28), the individual had 0.98 oz of total alcohol consumption. The corresponding current output peak 2405 was about 3488 picoamps. The corresponding blood alcohol concentration (BAC) is around 0.046 %. The corresponding peak signal width, for purpose of illustration, measured at half height of the peak, is 122 minutes. On day 5 (4/1), the individual had 0.98 oz of total alcohol consumption. The corresponding current output peak 2410 was about 2390 picoamps. The corresponding blood alcohol concentration (BAC) is around 0.043 %. The corresponding peak signal width is 138 minutes. On day 8 (4/4), the individual had 1.95 oz of total alcohol consumption. The corresponding current output peak 2415 was about 3430 picoamps. The corresponding blood alcohol concentration (BAC) is around 0.057 %. The corresponding peak signal width is 320 minutes. On day 12 (4/8), the individual had 0.82 oz of total alcohol consumption. The corresponding current output peak 2420 was about 1339 picoamps. The corresponding blood alcohol concentration (BAC) is around 0.015 %. The corresponding peak signal width is 80 minutes. On day 14 (4/10), the individual had 1.48 oz of total alcohol consumption. The corresponding current output peak 2425 was about 2650 picoamps. The corresponding blood alcohol concentration (BAC) is around 0.051 %. The corresponding peak signal width is 215 minutes.

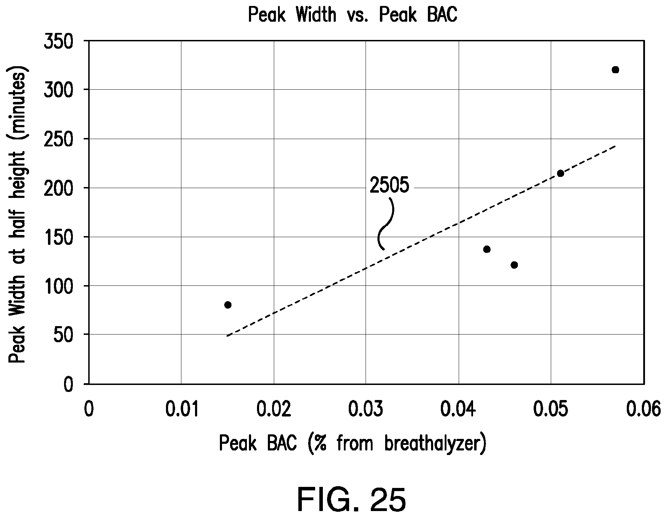

FIG. 25 is a diagram illustrating a correlation between the BAC measured at signal peaks and corresponding current peak widths. For purpose of illustration not limitation, a linear regression line 2505 is generated based on the exemplary data points as described above. For example and as described above, the BAC for subsequent peaks, including peaks effected by enzyme degradation of the alcohol sensor, can be determined using the following equation: $BAC = K1 \times K2 \times Width_{peak}$.

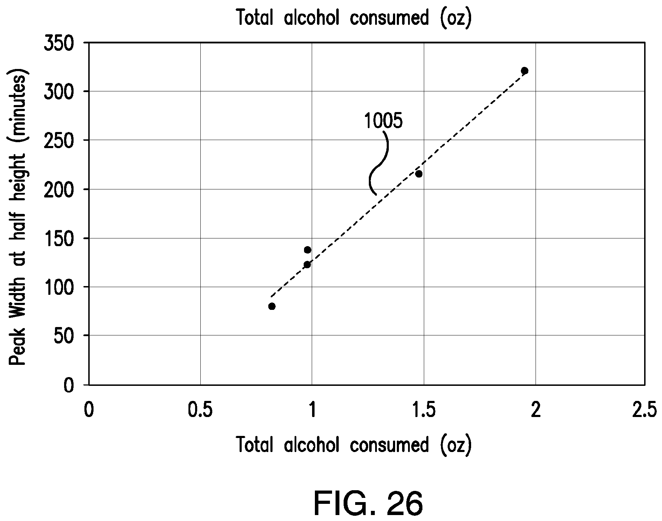

FIG. 26 is a diagram illustrating a correlation between the total alcohol consumptions at signal peaks and corresponding current peak widths. For purpose of illustration not limitation, a linear regression line 1005 is generated based on the exemplary data points as described above. For example and as described above, the total alcohol consumptions (TAC) and the peak widths are linearly correlated. As embodied herein, the total alcohol consumption can be determined using the following equation: $TAC = K3 \times Width_{peak}$, where K3 can be a predetermined coefficient.

For purpose of illustration only, and not limitation, alcohol sensors described herein can be used for various purposes, including but not limited to personal health monitoring, enforcing or monitoring compliance with alcohol-related rules or agreements, group therapy, and any other uses for information about alcohol levels, which can refer to a BAC as described herein, of a person or group. For example and without limitation, alcohol sensors can be used for self-monitoring by the user or remote monitoring by a caretaker or healthcare provider to allow the user to accurately monitor their alcohol intake over a period of time, for example and without limitation, to assist the user with identifying an unsafe amount of alcohol consumption and/or controlling alcohol consumption to a desired amount. As embodied herein, an alcohol sensor can be worn for a desired period, which can be any wear period described herein, and the results can be reported to the user for analysis and/or can be reported to the user's healthcare provider for consideration in advance of a consultation.

As embodied herein, for example only, alcohol sensors can be used to enforce compliance with alcohol-related conditions or restrictions of a criminal sentence parole, probation or diversion program by persons subject to such alcohol-related conditions or restrictions. As embodied herein, alcohol levels of the user can be sent to the parole, probation or diversion officer or other monitoring entity responsible for enforcing compliance with the terms of such programs. Additionally or alternatively, as embodied herein, alcohol sensors can be used to enforce compliance with occupational or workplace rules or regulations involving alcohol, for example, safety rules and regulations involving use of alcohol while or prior to operating a truck or other motor vehicle, machinery, or other heavy equipment, where alcohol levels of an employee can be communicated to the employer or other entity in charge of monitoring compliance with such rules or regulations. In addition, as embodied herein, alcohol sensors can be used to activate or disable external devices, for example, a motor vehicle, machinery, or other heavy equipment, can be activated upon confirmation using an alcohol sensor that the alcohol level of the user is sufficiently low for safe operation by the user, and/or can be locked or disabled if the alcohol sensor indicates the alcohol level of the user is unsafe for operation of the external device.

In addition, or as a further alternative, alcohol sensors can be used for group support for alcohol cessation. Members of a support group, which can be a formal or informal group of people interested in achieving or maintaining cessation of alcohol consumption, can each wear an alcohol sensor and agree to share, for example via a cloud-based system and monitoring application as described herein, alcohol sensor information with each other member of the support group. The alcohol sensor information can include the alcohol levels of the user and the status of the alcohol sensor as being active or working. Sharing the alcohol sensor information with the support group can encourage users to stay free of alcohol through peer support and can notify the support group when encouragement or an intervention with a member should be provided.

According to other aspects of the disclosed subject matter, alcohol sensors can be used to provide personalized insights to the user based on the alcohol level data. For purpose of illustration and not limitation, as embodied herein, alcohol level data can be correlated with an amount of dehydration of the user caused by alcohol intake. Alcohol level data can be used to determine a BAC of the user over time, for example and as described herein using a peak-width measurements of analyte levels over time. As embodied herein, the BAC of the user over time can be correlated, for example by a data reading device 120 or multi-purpose data receiving device 130, with an amount of dehydration of the user. The data reading device 120 or multi-purpose data receiving device 130 can be configured to provide a recommendation based on the amount of dehydration of the user determined from the alcohol level data. For example only, and without limitation, the data reading device 120 or multi-purpose data receiving device 130 can be configured to recommend the user consume a particular quantity of an oral electrolyte solution (e.g., Pedialyte® by Abbott Laboratories) based on the determined amount of dehydration. Such insights can be provided in combination with other data, including data from other analyte sensors. For example and without limitation, as embodied herein, a dual alcohol-ketone sensor can provide additional insights related to alcohol consumption. For example, a ketogenic diet can cause a person's BAC to increase faster, and thus, a data reading device 120 or multi-purpose data receiving device 130 in communication with a dual alcohol-ketone sensor (or separate alcohol and ketone sensors) can provide an indication to a user when a ketone analyte level or ketosis level of the user is in danger of causing or is causing the user's BAC to increase faster than when not in ketosis or at a lower ketone level.

In addition to the specific embodiments claimed below, the disclosed subject matter is also directed to other embodiments having any other possible combination of the dependent features claimed below and those disclosed above. As such, the particular features presented in the dependent claims and disclosed above can be combined with each other in other possible combinations. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the method and system of the disclosed subject matter without departing from the scope of the disclosed subject matter. Thus, it is intended that the disclosed subject matter include modifications and variations that are within the scope of the appended claims.

## Claims

1.   An apparatus comprising:
one or more processors (166) configured to:

receive a signal from an in vivo alcohol sensor (104), wherein at least a portion of the alcohol sensor is positioned in contact with a bodily fluid;
determine a peak signal width and a peak signal amplitude of the signal received from the alcohol sensor during an initial wear period, wherein the peak signal width is the amount of time over which the signal exceeds a background or threshold level of the signal; and
determine blood alcohol concentration by the equation:

$$BAC = K1 \times K2 \times Width_{peak,}$$

wherein BAC is the blood alcohol concentration, K1 is a factory sensitivity of the alcohol sensor determined from a calibration, K2 is a ratio of the peak signal amplitude to the peak signal width determined during the initial wear period, and $Width_{peak}$ is a peak signal width for a signal received from the alcohol sensor subsequent to the signal received from the alcohol sensor during the initial wear period.

2.   The apparatus of claim 1, wherein the initial wear period is 1, 2, 3, 4, or 5 days after insertion of the alcohol sensor.

3.   The apparatus of any of claims 1 or 2, wherein the alcohol sensor comprises one or more active areas, optionally wherein the one or more active areas include one or more enzymes, wherein the enzymes are configured to degrade over a life of the alcohol sensor.

4.   The apparatus of claim 3, wherein the one or more active areas of the alcohol sensor detect two or more analytes, wherein the two or more analytes include ethanol, glucose, or lactate.

5.   The apparatus of any of claims 1-4, wherein the one or more processor are further configured to:
display the blood alcohol concentration on the apparatus, wherein the apparatus is a reader device.

6.   The apparatus of any of claims 1-5, wherein the one or more processor are further configured to:

output an alert based on the blood alcohol concentration, wherein the alert is visual, auditory, or vibratory.

7. The apparatus of claim 6, wherein the alert comprises a recommendation to consume an oral electrolyte solution.

8. A method comprising:

receiving a signal from an in vivo alcohol sensor (104), wherein at least a portion of the alcohol sensor is positioned in contact with a bodily fluid;
determining a peak signal width and a peak signal amplitude of the signal received from the alcohol sensor during an initial wear period, wherein the peak signal width is the amount of time over which the signal exceeds a background or threshold level of the signal; and
determining blood alcohol concentration by the equation

$$BAC = K1 \ x \ K2 \ x \ Width_{peak}$$

wherein BAC is the blood alcohol concentration, K1 is a factory sensitivity of the alcohol sensor determined from a calibration, K2 is a ratio of the peak signal amplitude to the peak signal width determined during the initial wear period, and $Width_{peak}$ is a peak signal width for a signal received from the alcohol sensor subsequent to the signal received from the alcohol sensor during the initial wear period.

9. The method of claim 8, wherein the initial wear period is 1, 2, 3, 4, or 5 days after insertion of the alcohol sensor.

10. The method of any of claims 8 or 9, wherein the alcohol sensor comprises one or more active areas, optionally wherein the one or more active areas include one or more enzymes, wherein the enzymes are configured to degrade over a life of the alcohol sensor.

11. The method of claim 10, wherein the one or more active areas of the alcohol sensor detect two or more analytes, wherein the two or more analytes include ethanol, glucose, or lactate.

12. The method of any of claims 8-11, further comprising:
displaying the blood alcohol concentration on a reader device.

13. The method of any of claims 8-12, further comprising:
outputting an alert based on the blood alcohol concentration, wherein the alert is visual, auditory, or vibratory; optionally wherein the alert comprises a recommendation to consume an oral electrolyte solution.


**Patentansprüche**

1. Eine Vorrichtung, die Folgendes umfasst:
einen oder mehrere Prozessoren (166), die zu Folgendem ausgelegt sind:

Empfangen eines Signals von einem In-vivo-Alkoholsensor (104), wobei mindestens ein Anteil des Alkoholsensors in Kontakt mit einer Körperflüssigkeit positioniert ist;
Bestimmen einer Spitzensignalbreite und einer Spitzensignalamplitude des von dem Alkoholsensor während einer anfänglichen Trageperiode empfangenen Signals, wobei die Spitzensignalbreite die Zeitspanne ist, über die das Signal einen Hintergrund- oder Schwellenwert des Signals überschreitet; und
Bestimmen der Blutalkoholkonzentration durch die folgende Gleichung:

$$BAK = K1 \ x \ K2 \ x \ Breite_{Spitze,}$$

wobei BAK die Blutalkoholkonzentration ist, K1 eine werkseitige Empfindlichkeit des Alkoholsensors ist, die aus einer Kalibration bestimmt wurde, K2 ein Verhältnis der Spitzensignalamplitude zu der Spitzensignalbreite ist, die während der anfänglichen Trageperiode bestimmt wurden, und $Breite_{Spitze}$ eine Spitzensignalbreite für ein Signal ist, das von einem Alkoholsensor empfangen wurde, nachdem das Signal von dem Alkoholsensor während der anfänglichen Trageperiode empfangen wurde.

2. Vorrichtung nach Anspruch 1, wobei die anfängliche Trageperiode 1, 2, 3, 4 oder 5 Tage nach dem Einsetzen des Alkoholsensors beträgt.

3. Vorrichtung nach einem der Ansprüche 1 oder 2, wobei der Alkoholsensor einen oder mehrere aktive Bereiche umfasst, wobei der eine oder die mehreren aktiven Bereiche optional ein oder mehrere Enzyme einschließen, wobei die Enzyme dazu ausgelegt sind, über eine Lebensdauer des Alkoholsensors abgebaut zu werden.

4. Vorrichtung nach Anspruch 3, wobei der eine oder die mehreren aktiven Bereiche des Alkoholsensors zwei oder mehr Analyten nachweisen, wobei die zwei oder mehr Analyten Ethanol, Glucose oder Lactat einschließen.

5. Vorrichtung nach einem der Ansprüche 1-4, wobei der eine oder die mehreren Prozessoren ferner zu Folgendem ausgelegt sind:
Anzeigen der Blutalkoholkonzentration auf der Vorrichtung, wobei die Vorrichtung ein Lesegerät ist.

6. Vorrichtung nach einem der Ansprüche 1-5, wobei der eine oder die mehreren Prozessoren ferner zu Folgendem ausgelegt sind:
Ausgeben einer auf der Blutalkoholkonzentration basierenden Warnmeldung, wobei die Warnmeldung visuell, akustisch oder vibratorisch ist.

7. Vorrichtung nach Anspruch 6, wobei die Warnmeldung eine Empfehlung zum Einnehmen einer oralen Elektrolytlösung umfasst.

8. Ein Verfahren, das Folgendes umfasst:

Empfangen eines Signals von einem In-vivo-Alkoholsensor (104), wobei mindestens ein Anteil des Alkoholsensors in Kontakt mit einer Körperflüssigkeit positioniert ist;
Bestimmen einer Spitzensignalbreite und einer Spitzensignalamplitude des von dem Alkoholsensor während einer anfänglichen Trageperiode empfangenen Signals, wobei die Spitzensignalbreite die Zeitspanne ist, über die das Signal einen Hintergrund- oder Schwellenwert des Signals überschreitet; und
Bestimmen der Blutalkoholkonzentration durch die folgende Gleichung:

$$BAK = K1 \times K2 \times Breite_{Spitze},$$

wobei BAK die Blutalkoholkonzentration ist, K1 eine werkseitige Empfindlichkeit des Alkoholsensors ist, die aus einer Kalibration bestimmt wurde, K2 ein Verhältnis der Spitzensignalamplitude zu der Spitzensignalbreite ist, die während der anfänglichen Trageperiode bestimmt wurden, und $Breite_{Spitze}$ eine Spitzensignalbreite für ein Signal ist, das von einem Alkoholsensor empfangen wurde, nachdem das Signal von dem Alkoholsensor während der anfänglichen Trageperiode empfangen wurde.

9. Verfahren nach Anspruch 8, wobei die anfängliche Trageperiode 1, 2, 3, 4 oder 5 Tage nach dem Einsetzen des Alkoholsensors beträgt.

10. Verfahren nach einem der Ansprüche 8 oder 9, wobei der Alkoholsensor einen oder mehrere aktive Bereiche umfasst, wobei der eine oder die mehreren aktiven Bereiche optional ein oder mehrere Enzyme einschließen, wobei die Enzyme dazu ausgelegt sind, über eine Lebensdauer des Alkoholsensors abgebaut zu werden.

11. Verfahren nach Anspruch 10, wobei der eine oder die mehreren aktiven Bereiche des Alkoholsensors zwei oder mehr Analyten nachweisen, wobei die zwei oder mehr Analyten Ethanol, Glucose oder Lactat einschließen.

12. Verfahren nach einem der Ansprüche 8-11, das ferner Folgendes umfasst:
Anzeigen der Blutalkoholkonzentration auf einem Lesegerät.

13. Verfahren nach einem der Ansprüche 8-12, das ferner Folgendes umfasst:
Ausgeben einer auf der Blutalkoholkonzentration basierenden Warnmeldung, wobei die Warnmeldung visuell, akustisch oder vibratorisch ist; wobei die Warnmeldung optional eine Empfehlung zum Einnehmen einer oralen Elektrolytlösung umfasst.

**Revendications**

1. Appareil comprenant :
   un ou plusieurs processeurs (166) configurés pour :

   recevoir un signal en provenance d'un capteur d'alcool in vivo (104), au moins une partie du capteur d'alcool étant positionnée en contact avec un fluide corporel ;
   déterminer une largeur de signal de crête et une amplitude de signal de crête du signal reçu en provenance du capteur d'alcool au cours d'une période de port initiale, la largeur de signal de crête étant la durée pendant laquelle le signal dépasse un niveau de fond ou de seuil du signal ; et
   déterminer le taux d'alcoolémie à l'aide de l'équation :

$$BAC = K1 \text{ x } K2 \text{ x } Largeur_{crête},$$

   dans laquelle BAC est le taux d'alcoolémie, K1 est une sensibilité d'usine du capteur d'alcool déterminée à partir d'un étalonnage, K2 est un rapport de l'amplitude de signal de crête à la largeur de signal de crête déterminée durant la période de port initiale, et
   $Largeur_{crête}$ est une largeur de signal de crête pour un signal reçu en provenance du capteur d'alcool après le signal reçu en provenance du capteur d'alcool durant la période de port initiale.

2. Appareil selon la revendication 1, la période de port initiale étant de 1, 2, 3, 4 ou 5 jours après l'insertion du capteur d'alcool.

3. Appareil selon l'une quelconque des revendications 1 ou 2, dans lequel le capteur d'alcool comprend une ou plusieurs zones actives, optionnellement dans lequel les une ou plusieurs zones actives comprennent une ou plusieurs enzymes, les enzymes étant configurées pour se dégrader au cours d'une durée de vie du capteur d'alcool.

4. Appareil selon la revendication 3, dans lequel les une ou plusieurs zones actives du capteur d'alcool détectent deux analytes ou plus, les deux analytes ou plus comprenant l'éthanol, le glucose, ou un lactate.

5. Appareil selon l'une quelconque des revendications 1 à 4, dans lequel les un ou plusieurs processeurs sont en outre configurés pour :
   afficher le taux d'alcoolémie sur l'appareil, l'appareil étant un dispositif de lecture.

6. Appareil selon l'une quelconque des revendications 1 à 5, dans lequel les un ou plusieurs processeurs sont en outre configurés pour :
   sortir une alerte basée sur le taux d'alcoolémie, l'alerte étant visuelle, auditive ou vibratoire.

7. Appareil selon la revendication 6, dans lequel l'alerte comprend une recommandation de consommer une solution orale d'électrolytes.

8. Procédé comprenant :

   la réception d'un signal en provenance d'un capteur d'alcool in vivo (104), au moins une partie du capteur d'alcool étant positionnée en contact avec un fluide corporel ;
   la détermination d'une largeur de signal de crête et d'une amplitude de signal de crête du signal reçu en provenance du capteur d'alcool au cours d'une période de port initiale, la largeur de signal de crête étant la durée pendant laquelle le signal dépasse un niveau de fond ou de seuil du signal ; et
   la détermination du taux d'alcoolémie à l'aide de l'équation :

$$BAC = K1 \text{ x } K2 \text{ x } Largeur_{crête}$$

   dans laquelle BAC est le taux d'alcoolémie, K1 est une sensibilité d'usine du capteur d'alcool déterminée à partir d'un étalonnage, K2 est un rapport de l'amplitude de signal de crête à la largeur de signal de crête déterminée durant la période de port initiale, et
   $Largeur_{crête}$ est une largeur de signal de crête pour un signal reçu en provenance du capteur d'alcool après le signal reçu en provenance du capteur d'alcool durant la période de port initiale.

9. Procédé selon la revendication 8, dans lequel la période de port initiale est de 1, 2, 3, 4 ou 5 jours après l'insertion du capteur d'alcool.

10. Procédé selon l'une quelconque des revendications 8 ou 9, dans lequel le capteur d'alcool comprend une ou plusieurs zones actives, optionnellement dans lequel les une ou plusieurs zones actives comprennent une ou plusieurs enzymes, les enzymes étant configurées pour se dégrader au cours d'une durée de vie du capteur d'alcool.

11. Procédé selon la revendication 10, dans lequel les une ou plusieurs zones actives du capteur d'alcool détectent deux analytes ou plus, les deux analytes ou plus comprenant l'éthanol, le glucose ou un lactate.

12. Procédé selon l'une quelconque des revendications 8 à 11, comprenant en outre :
l'affichage du taux d'alcoolémie sur un dispositif de lecture.

13. Procédé selon l'une quelconque des revendications 8 à 12, comprenant en outre :
la sortie d'une alerte basée sur le taux d'alcoolémie, l'alerte étant visuelle, auditive ou vibratoire ; optionnellement dans lequel l'alerte comprend une recommandation de consommer une solution orale d'électrolytes.

FIG. 1A

100a

Data Receiving
Device 120

User Device 140

Remote
Application
Server 150

Sensor 110

Multi-Purpose Data
Receiving Device 130

Application
Storefront Server 160

FIG. 1B

122 — | Display |

121 — ( Input Component )

Reader Device
120

222 —

Communications
Processor

223 —

| Memory |

224 —

Applications
Processor

225 —

| Memory |

— 206

229 —

228 —

| RF Transceiver |

230 —

| Memory |

Multi-Functional
(WiFi)
(NFC)
(Bluetooth, BTLE)
(GPS)

— 234

| Power Supply |

| Power Management |

226

238

232

FIG. 2A

FIG. 2B

Sensor Electronics
160

161

ASIC

104

162

Analyte Sensor

AFE

Power Mgmt.
Circuitry

164

170

Power Source

168

Communication
Circuitry

Processor

166

171

163

Memory

102
Sensor Control
Device

FIG. 2C

Sensor Electronics
160

104

163

162

174

Analyte Sensor

AFE

Chip

Memory

165

Memory

Power Mgmt.
Circuitry

164

161
ASIC

171

168

Communication
Circuitry

Processor

166

170

Power Source

FIG. 2D

102
Sensor Control Device

110
Sensor

5040
Communication Module

5041
BLE

5043
Memory

5050
Power Module

5060
Sensing Hardware

5000
ASIC

5010
Microcontroller

5020
Memory

5030
Storage

5025
NFC

FIG. 2E

FIG. 3A

FIG. 3B

FIG. 3C

FIG. 3D

FIG. 3E

FIG. 3F

FIG. 4A

FIG. 4B

FIG. 4C

FIG. 4E

FIG. 4D

FIG. 4F

20150

20702

201102

20704

205612

20701

205014

20710

20102

20708

# FIG. 4G

FIG. 6A

FIG. 5

FIG. 6B

EP 4 262 555 B1

FIG. 7A

FIG. 7B

FIG. 8A

FIG. 8B

FIG. 8C

FIG. 9B

FIG. 9A

FIG. 10A

FIG. 10B

FIG. 11A

FIG. 11B

FIG. 11C

FIG. 12B

FIG. 12A

FIG. 12C

**FIG. 13A**

FIG. 13B

FIG. 13C

FIG. 13D

1120

1108

1030

1102

FIG. 13E

216

222

# FIG. 13F

FIG. 14

FIG. 15

FIG. 16

FIG. 17

1820    1818                    1800

1814
1820                    1812    1816

# FIG. 18A

FIG. 18B

FIG. 18C

FIG. 19

FIG. 20

FIG. 21A

FIG. 21B

**FIG. 22**

**FIG. 23**

FIG. 24

FIG. 25

FIG. 26

**REFERENCES CITED IN THE DESCRIPTION**

**Patent documents cited in the description**

- US 63127848
- US 2020237276 A
- US 20130150691 A
- US 20210204841 A
- WO 2018136898 A
- WO 2019236850 A
- WO 2019236859 A
- WO 2019236876 A
- US 20200196919 A
- US 20160331283 A
- US 20180235520 A
- US 20140171771 A
- US 201000230285 A
- US 20190274598 A